# DEMANDE DE BREVET EUROPEEN

(11) **EP 0 957 170 A1**
(43) Date de publication de la demande: **17.11.1999**
(21) Numéro de dépôt: 98201312.0
(22) Date de dépôt: 22.04.1998
(51) Int. Cl.: C12N 15/52, C12P 19/18, C12N 9/10, C08B 37/00, C12Q 1/68, C12N 1/21

(54) **Identification de gènes de Lactobacillus delbrueckii subsp bulgaricus Lfi5 impliqués dans la biosynthèse d'exopolysaccharides**

(71) Demandeur: SOCIETE DES PRODUITS NESTLE S.A., 1800 Vevey (CH)
(72) Inventeur: Lamothe, Gilbert, 1012 Lausanne (CH); Stingele, Francesca, 1018 Lausanne (CH)
(74) Mandataire: Van Malderen, Michel

(57) **Abrégé**

Enzyme recombinée, susceptible d'être impliquée dans la biosynthèse de l'EPS ayant l'unité saccharidique répétitive 
β-D-Gal*p*          α-L-Rha*p*          β-D-Gal*p*
1                    1                    1
|                    |                    |
4                    3                    3
→3)-β-D-Gal*p*(1→4)-α-D-Gal*p*(1→2)-α-D-Gal*p*(1→3)-β-D-Glc*p*(1→
 catalysant spécifiquement l'une des liaisons suivantes entre sucres: (1) la liaison osidique β-1,3 entre le carbone 1 d'un D-Gal*p* activé et le carbone 3 du sucre présent à l'extrémité non-réductrice d'une chaîne saccharidique en formation; (2) la liaison osidique β-1,4 entre le carbone 1 d'un D-Gal*p* activé et le carbone 4 du sucre présent à l'extrémité non-réductrice d'une chaîne saccharidique en formation; (3) la liaison osidique α-1,3 entre le carbone 1 d'un L-Rha*p* activé et le carbone 3 du sucre présent à l'extrémité non-réductrice d'une chaîne saccharidique en formation; lesdites chaînesétant composées notamment d'une chaîne principale de 1 à 4 D-Gal*p* et/ou D-Glc*p* liés entre-eux par des liaisons osidiques α-1,2, α-1,3, β-1,4 et/ou β-1-3, et lesdits sucres présents aux extrémités non-réductrices étant liés à un seul autre sucre par son carbone 1. L'invention concerne aussi tout ADN recombinant codant pour une enzyme recombinée selon l'invention, ainsi que toutes cellules comprenant, intégrées dans son génome ou par le moyen d'un plasmide réplicable, un ADN recombiné, et exprimant une enzyme recombinée fonctionnelle selon l'invention. Enfin l'invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

## Description

La présente invention se rapporte à de nouvelles fonctions enzymatiques de bactéries lactiques alimentaires, ainsi que de nouveaux enzymes et gènes, impliqués dans la biosynthèse d'exopolysaccharides.

### État de la technique

Il est connu que les bactéries lactiques sont susceptibles de produire dans leur milieu de culture deux classes de polysaccharides, à savoir les homopolysaccharides comme les dextranes ou les levanes qui sont constitués par l'assemblage répété d'un seul sucre, et les hétéropolysaccharides appelés communément exopolysaccharides ou EPS (EPS est l'abréviation du terme "exopolysaccharide") constitués par l'assemblage de plusieurs sucres différents formant une unité répétitive (Cerning J., Bactéries lactiques, Vol I, de Rossart H et Luquet F. M., Lorica, 309-329, 1994).

Une bactérie lactique alimentaire produisant un EPS peut conférer un caractère filant et/ou une texture lisse et crémeuse à un lait acidifié (Cerning *et al*., FEMS Microbiol., 87, 113-130, 19/90). Les EPS peuvent aussi présenter des activités biologiques particulièrement intéressantes pour la santé humaine ou animale, comme des activités anti-tumeurs ou probiotiques, par exemple (Oda M. *et al*., Agric. Biol. Chem., 47, 1623-1625, 1983; EP94870139.6)

Par ailleurs, l'industrie alimentaire est confrontée à une instabilité génétique de la biosynthèse des EPS dans les bactéries lactiques. Ceci se traduit généralement au cours d'une fermentation par la perte de la production d'EPS par tout ou partie des bactéries lactiques (voir "Cerning J." ci-dessus). Les produits fermentés industriels sont ainsi sujets à des variations dans leur contenu en EPS, ce qui n'est pas toujours acceptable. Pour remédier à ces problèmes, l'industrie recours actuellement à l'isolement et la caractérisation périodique de ses bactéries de manière à séparer celles qui ont perdu leur caractère originel.

On connaît des gènes de bactéries lactiques alimentaires impliqués dans la biosynthèse d'EPS.

WO92/02142 révèle ainsi l'existence du plasmide pHV67 qui produit dans *Lactococcus lactis* subsp. *lactis* (mésophile) une substance capable d'augmenter la viscosité d'un lait fermenté. La structure saccharidique de cette substance n'étant pas connue, les différentes fonctions des enzymes codées par pHV67 sont également inconnues.

US5066588 décrit deux plasmides provenant d'une souche de *Streptococcus cremoris* (mésophile) capable de conférer un caractère épaississant à un *Streptococcus lactis*. La structure saccharidique de cet épaississant n'étant pas connue, les différentes fonctions des enzymes codées par ces plasmides sont également inconnues.

Vescovo *et al*. ont mis en évidence un plasmide d'une souche *Lactobacillus casei* subsp. *casei* (mésophile) codant pour un phénotype Muc+, c'est à dire pour des fonctions liées à la production d'épaississants exocellulaires (Vescovo *et al*., Biotechnology Letters, Vol II, 709-712, 1989). La structure saccharidique de cet épaississant n'étant pas connue, les différentes fonctions des enzymes codées par ce plasmide sont également inconnues.

EP750043 (Société des Produits Nestlé) décrit un opéron de gènes de la souche *Streptococcus thermophilus* CNCM 1-1590 impliqué dans la synthèse d'un EPS ayant la structure répétitive suivante.

→3)-β-D-Glcp(1→3)-α-D-GalpNAc(1→3)-β-D-Galp(1→
6
↑
1
α -D-Galp

Des travaux menés sur ces enzymes (résultats non-publiés) ont permis de montrer que la biosynthèse de cet EPS se fait de manière progressive avec, à chaque étape, l'addition d'une nouvelle unité de sucre qui vient s'attacher par sa fonction semi-acétalique à un hydroxyle alcoolique d'une autre unité de sucre, laquelle est à l'extrémité d'une chaîne de sucres liée à une amorce. Ces enzymes catalysent ou contrôlent ainsi spécifiquement les fonctions suivantes.
(1) La liaison sous forme d'un isomère α ou β entre le carbone 1 (qui porte la fonction réductrice aldéhyde) d'un D-Gal*p* activé et un phosphate d'une amorce lipophilique notamment du genre undécaprénol, ou protéique notamment du genre ACP (Acyl Carrier Protein), par exemple.
(2) La liaison osidique α-1,3 entre le carbone 1 d'un Gal*p*NAc activé (UDP-Gal*p*NAc) et le carbone 3 du D-Gal*p* lié à son amorce.
(3) La liaison osidique β-1,3 entre le carbone 1 d'un D-Glc*p* activé (UDP-D-Glc*p*) et le carbone 3 du sucre présent à l'extrémité non-réductrice de la chaîne β-D-Gal*p*(1→3)-α/β-D-Gal*p*-amorce.
(4) La liaison osidique α-1,6 entre le carbone 1 d'un D-Gal*p* activé et le carbone 6 du sucre présent à l'extrémité non-réductrice de la chaîne β-D-Glc*p*(1→3)β-D-Gal*p*(1→3)α/β-D-Gal*p*-amorce.
(5) Le transport des unités saccharidiques répétitives de l'EPS décrit ci-dessus à l'extérieur de la cellule bactérienne.
(6) La liaison osidique β-1,3 entre les unités saccharidiques répétitives de l'EPS ci-dessus.
(7) La régulation du nombre d'unités saccharidiques dans l'EPS, et donc de son poids moléculaire final.

Van Kranenburg *et al*. ont aussi décrit un opéron de gènes, présent sur un plasmide d'une souche *Lactococcus lactis*, impliqué dans la synthèse d'un EPS ayant la structure répétitive suivante (Mol. Microbiology, 24, 387-397, 1997).

→4)-β-D-Gal*p*(1→4)-β-D-Glc*p*(1→4)-β-D-Glc*p*(1→
2 3
↑ ↑
1 PO4
α-L-Rha*p* ↑
1
α-D-Gal*p*

Bien que les enzymes codant pour cet EPS soient maintenant connues, il reste encore à élucider la spécificité de chaque enzyme. Pour le moment on peut valablement supposer, au regard des homologies avec d'autres opérons impliqués dans la biosynthèse de polysaccharides, que la biosynthèse débute par la liaison d'un Glc*p* activé sur une amorce lipophilique ou protéique.

Les connaissances relatives à la biosynthèse des EPS dans les bactéries lactiques alimentaires sont encore néanmoins très fragmentaires. Cependant il est maintenant admis que, basée sur les structures d'EPS élucidées jusqu'à présent et par analogie avec la production de composés tels que les lipopolysaccharides (Whitfield *et al*., Adv. Microbiol. Physiol., 35, 136-246, 1993), la synthèse débute par l'activation de monomères de sucres (glucose, galactose...) en sucres nucléotidiques utilisés comme précurseurs par les glycosyltransférases. La première étape de la biosynthèse de l'unité répétitive est catalysée par une glycosyltransférase particulière qui reconnaît, d'une part, un précurseur de type sucre nucléotidique et, d'autre part, une amorce lipophilique, notamment du genre undécaprénol-phosphate, ou protéique notamment du genre ACP, par exemple. Les autres glycosyltransférases agissent ensuite séquentiellement en ajoutant un sucre spécifique sur l'unité répétitive en construction. Les unités répétitives complètes sont ensuite exportées vers le milieu extracellulaire avant d'être polymérisées.

A ce jour, il existe toujours un besoin de disposer de nouvelles enzymes impliquées dans la synthèse d'EPS, et notamment des enzymes catalysant des liaisons spécifiques entre des sucres. La présente invention vise à remplir ces besoins.

### Résumé de l'invention

A cet effet, la présente invention concerne toute enzyme recombinée, que l'on peut purifier et qui est susceptible d'être impliquée dans la biosynthèse de l'EPS ayant l'unité saccharidique répétitive suivante, 
β-D-Gal*p*          α-L-Rha*p*          β-D-Gal*p*
1                    1                    1
|                    |                    |
4                    3                    3
→3)-β-D-Gal*p*(1→4)-α-D-Gal*p*(1→2)-α-D-Gal*p*(1→3)-β-D-Glc*p*(1→
 et catalysant spécifiquement:
- la liaison osidique β-1,3 entre le carbone 1 d'un D-Gal*p* activé et le carbone 3 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique β-1,4 entre le carbone 1 d'un D-Gal*p* activé et le carbone 4 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique α-1,3 entre le carbone 1 d'un L-Rha*p* activé et le carbone 3 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1.

Un autre objet de l'invention concerne tout ADN recombiné codant pour une enzyme selon l'invention, et notamment tout vecteur d'ADN ou toute cellule renfermant un tel ADN recombiné, et exprimant une enzyme recombinée fonctionnelle.

Un autre objet de l'invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

Enfin, un dernier objet de la présente invention concerne l'utilisation d'une enzyme recombinée selon l'invention, ou d'un ADN selon l'invention, pour la synthèse d'un EPS.

### Description de la figure

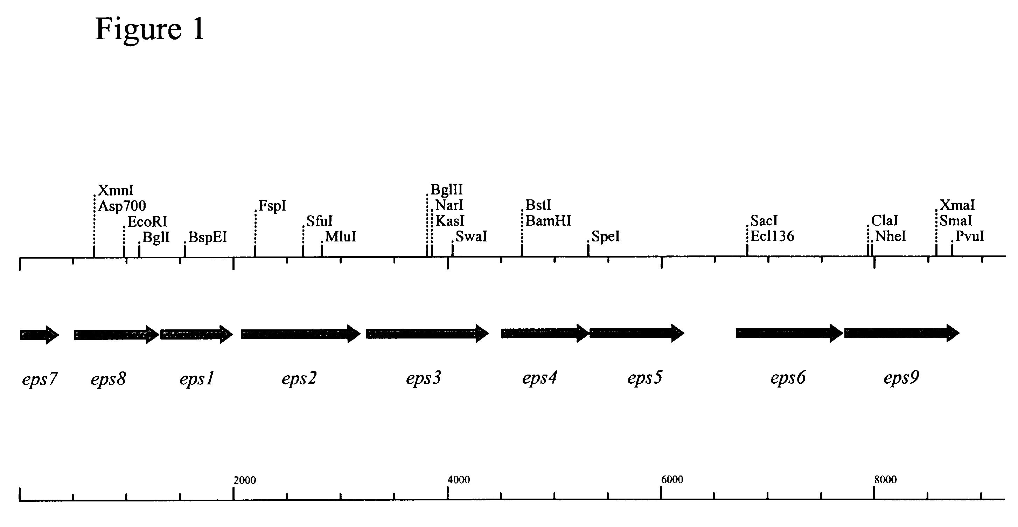

La figure 1 représente la carte physique de l'opéron impliqué dans la synthèse de l'EPS de la souche *S. thermophilus* CNCM I-800. Les flèches horizontales indiquent la présence de cadres de lectures (ORF) potentiels. Les noms des gènes correspondants aux ORFs sont indiqués en dessous des flèches.

### Description détaillée de l'invention

Dans la suite de la description, les séquences en nucléotides des gènes ou amorces utilisés, ainsi que les séquences en acides aminés des enzymes recombinées, sont représentées dans la liste de séquence fournie ci-après, et sont nommées, pour des raisons de simplification, sous les sigles numérotés "SEQ ID NO:".

Le terme "EPS" désigne un polymère formé par la condensation d'un grand nombre de molécules de sucres (= oses) de types différents. Cet EPS est plus particulièrement constitué par l'assemblage répété d'une seule unité saccharidique, elle-même formée d'une chaîne principale de sucres dont les extrémités sont impliquées dans la polymérisation des unités. Cette chaîne principale peut comprendre en outre des ramifications de sucres qui ne sont pas impliquées dans la polymérisation des unités, lesdites ramifications étant greffées par des liaisons osidiques sur un ou plusieurs sucres de cette chaîne.

On représente une unité saccharidique en indiquant (1) les sigles usuels des sucres (Glc: glucose; Gal: galactose; Rha: rhamnose; etc.) ; (2) le type de chaque liaison osidique sous la forme d'une flèche entre les numéros de carbone de deux sucres adjacents (les numéros de carbone attribués sur le cycle d'un sucre étant ceux reconnus par la nomenclature internationale des sucres: IUPAC) ; (3) l'anomérie de chaque liaison osidique sous la forme du sigle α ou β, ledit sigle étant positionné à gauche d'un sucre pour indiquer l'anomérie de la liaison osidique présente à la droite de ce sucre; (4) la nature pyranose ou furanose de chaque sucre en adjoignant la lettre *p* ou *f* juste après le sigle de chaque sucre ; (5) et la série D ou L de chaque sucre, en positionnant la lettre à gauche du sucre concerné.

Dans une chaîne saccharidique en formation, la réaction directe entre une fonction semi-acétalique d'une nouvelle unité de sucre et un hydroxyle alcoolique du sucre présent à l'extrémité non-réductrice de la chaîne ne se fait spontanément, et c'est pourquoi le groupement semi-acétalique doit au préalable être activé, c'est-à-dire rendu plus réactif en vue de la formation de la liaison osidique. Cette activation est catalysée par des enzymes bactériennes formant un sucre nucléotidique, et plus particulièrement un uridine-diphosphate de D-Glc*p* (UDP-Glc*p*) ou D-Gal*p* (UDP-Gal*p*), ou une thymidine-diphosphate de L-Rha*p* (TDP-Rha*p*) dans le contexte de la présente invention. Pour initier la formation d'un chaîne saccharidique, un premier sucre activé doit d'abord être lié sur le phosphate d'une amorce lipophilique ou protéique. L'addition d'une nouvelle unité de sucre activé sur la chaîne se fera ensuite par sa fonction semi-acétalique au niveau d'un hydroxyle alcoolique du sucre présent à l'extrémité non-réductrice de cette chaîne, c'est-à-dire à l'extrémité opposée de celle où l'amorce est fixée.

Dans la suite de la description, on représente une chaîne saccharidique en formation, en indiquant les sigles usuels des sucres, les types de liaisons osidiques (y compris l'anomérie), la position de l'amorce sur la chaîne, la nature du cycle et de la série D ou L de chaque sucre. Du fait que l'on ne sait pas encore quel isomère est formé lorsque le premier sucre d'une chaîne est lié à une amorce, par commodité d'écriture, on utilise aussi le sigle "α/β" afin d'indiquer que l'un des deux isomères est formé. L'emplacement d'une ramification sur une chaîne saccharidique peut être également indiqué dans une chaîne par la présence de crochets à l'intérieur desquels les sucres, les types de liaisons osidiques et la nature du cycle et de la série D ou L de chaque sucre sont représentés, chaque crochet étant positionné à gauche du sucre sur lequel la ramification est greffée.

Au sens de la présente invention, on entend par "séquence homologue" toute séquence nucléotidique ou d'acides aminés, conduisant ou donnant un variant d'enzyme qui catalyse l'assemblage de sucres, ne différant des séquences selon l'invention que par la substitution, la délétion ou l'addition d'un petit nombre de bases nucléotidiques ou d'acides aminés, par exemple 1 à 150 paires de bases (pb) ou 1 à 50 acides aminés. Ces variants d'enzyme peuvent conserver la même spécificité d'assemblage de sucres que les enzymes dont ils dérivent. La substitution, la délétion ou l'addition d'un petit nombre d'acides aminés peut cependant aussi conduire à changer la spécificité enzymatique.

La sélection d'une séquence homologue est considérée comme évidente pour l'homme du métier, puisque l'on peut facilement créer des variants d'enzymes selon l'invention, en mettant en oeuvre des méthodes de mutagénèse classiques, et notamment en adaptant les méthodes décrites par Adams *et al*. (EP402450; Genencor), par Dunn *et al*. (Protein Engineering, 2, 283-291, 1988), par Greener *et al*. (Strategies, 7, 32-34, 1994) et/ou par Deng *et al*. (Anal. Biochem, 200, 81, 1992), par exemple.

Dans ce cadre, on considérera en particulier comme homologues deux séquences d'ADN qui, du fait de la dégénérescence du code génétique, codent pour un même polypeptide. De même, on peut aussi considérer comme homologue deux séquences d'ADN s'hybridant dans des conditions stringentes, c'est-à-dire s'hybridant toujours après une étape d'hybridation à 65°C pendant 15h dans un tampon d'hybridation (SSPE 1,5x: 0,225 M de NaCl, 0,0015 M d'EPTA, 0,015 M de NaH₂PO₄, pH7 ; 1% de SDS et 1% de lait déshydraté), suivie de 6 étapes successives de lavage à 65°C dans un tampon comprenant différentes dilutions de SSC 10x (1,5 M de NaCl, 0,15 M de citrate de sodium, pH7) et 0,1% SDS, respectivement pendant 2 fois 10 min avec du SSC 2x, pendant 2 fois 10 min avec du SSC 1x, et pendant 2 fois 5 min avec du SSC 0,1x.

On peut aussi considérer comme homologues les séquences d'ADN présentant plus de 50% d'identité avec les séquences selon l'invention, en particulier plus de 70%, l'identité étant déterminée par le rapport entre le nombre de bases nucléotidiques d'une séquence homologue qui sont identiques à celles d'une séquence selon l'invention, et le nombre total de bases nucléotidiques de ladite séquence selon l'invention, par exemple.

De même, on considérera comme homologues deux protéines fonctionnelles qui sont reconnues par un même anticorps, le rapport des valeurs d'intensité de reconnaissance des deux protéines par l'anticorps n'excédant pas 1000 dans un test ELISA, de préférence 100, par exemple. On considérera plus particulièrement comme homologues les séquences en acides aminés qui présentent plus de 70% d'identité avec les séquences selon l'invention, en particulier plus de 80% ou 90%. Dans ce dernier cas, l'identité est déterminée par le rapport entre le nombre d'acides aminés d'une séquence homologue qui sont identiques à celles d'une séquence selon l'invention, et le nombre total d'acides aminés de ladite séquence selon l'invention.

Pour isoler un ADN recombiné selon la présente invention, il est possible de constituer une banque de grands fragments d'ADN d'une bactérie lactique produisant un EPS dans une bactérie lactique ne produisant pas d'EPS, puis de sélectionner le ou les clone(s) produisant un EPS. Pour cela, on digère l'ADN génomique d'une bactérie lactique produisant un EPS par une enzyme de restriction qui est spécifique d'un site de restriction relativement rare (*Bam*HI, *Sal*I, *Pst*I) ou par une digestion partielle avec *Sau*3A, par exemple. On clone le produit de digestion dans un plasmide d'expression ou d'intégration qui accepte de grands fragments (plasmide pSA3, Dao *et al*., Appl. Environ. Microbiol., 49, 115-119, 1985), on introduit les plasmides recombinants dans une espèce identique ou proche ne produisant pas d'EPS, telle que *Lactobacillus helveticus* ou *Lactococcus lactis*. Enfin on sélectionne au moins un clone transformé produisant un EPS, puis on identifie, on isole et on séquence classiquement le fragment d'ADN responsable de la production d'EPS.

Vu que les ADN recombinés selon la présente invention sont susceptibles d'être de grande taille, du fait qu'ils peuvent contenir un groupe de gènes impliqués dans la biosynthèse d'EPS, on peut préférer introduire les plasmides recombinants dans la même souche de bactérie lactique dont provienne les fragments, à la différence près que cette souche a perdu la capacité de produire des EPS suite à un traitement mutagénique (traitement U.V., chimique ou par transposon).

Une autre alternative consiste à préparer des amorces nucléotidiques dérivées de gènes connus impliqués dans la biosynthèse d'un EPS, puis à réaliser une PCR sur l'ADN génomique d'une bactérie lactique produisant un EPS et pour laquelle on désire identifier les gènes impliqués dans la biosynthèse de cet EPS. Cette technique est cependant excessivement difficile à mettre en oeuvre, car le choix de la séquence nucléotidique des amorces sera déterminante dans l'isolement d'un gène. Puisque l'on ne connaît pas d'avance les homologies entre les gènes recherchés et les gènes connus, ce choix procède d'une démarche inventive, de par la nécessaire sélection opérée parmi une multiplicité d'amorces possibles.

Quelques critères peuvent cependant être retenus, tel que (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codées naturellement par 1 ou 2 codons, tel que la méthionine, le tryptophane, la glutamine ou l'acide glutamique, par exemple ; etc.

Il faut remarquer que les méthodes de sélection décrites brièvement ci-dessus peuvent être appliquées à toutes les bactéries lactiques connues, notamment aux bactéries lactiques mésophiles comme par exemple *Streptococcus cremoris*, *Streptococcus lactis*, *Lactobacillus casei* subsp. *casei* et *Lactobacillus sake*, et les bactéries lactiques thermophiles comme par exemple *Streptococcus thermophilus*, *Lactobacillus delbruecki* subsp. *bulgaricu*s et *Lactobacillus helveticus*. A cet effet, l'homme du métier dispose de techniques de transformation pour chaque espèce de bactérie lactique, et en particulier pour *Lactobacillus delbruecki* subsp. *bulgaricus* (Sasaki Y. *et al*., FEMS Microbiology Reviews, 12, Fourth Symposium on Lactic Acid Bacteria, Noodwijkerhout, The Netherlands, Sept 1993; Satoh *et al*., Appl. Env. Microb., 63, 4593-4596, 1997).

De plus, les méthodes de sélection décrites ci-dessus permettent le plus souvent d'isoler seulement une partie d'un gène ou d'un groupe de gènes impliqués dans la biosynthèse d'un EPS. Néanmoins, l'homme du métier peut facilement identifier la partie restante du gène ou du groupe de gènes en sélectionnant dans une banque chromosomique, à l'aide de sondes nucléiques basées sur un fragment isolé, un ou plusieurs clones renfermant la partie restante, par exemple.

On a pu ainsi caractériser une séquence d'ADN de 9,2 kb de la souche *Lactobacillus delbrueckii bulgaricus* Sfi5 déposée le 4 octobre 1988 selon le traité de Budapest, auprès de la Collection Nationale de Culture de Microorganisme (C.N.C.M.), 28 rue du Docteur Roux, 75724 Paris cedex 15, France, où elle a reçu le numéro de dépôt CNCM I-800. Par ailleurs, cette souche Gram-positif présente au microscope un aspect de bâtonnets non-flagellés. Cette souche est non-sporulante et anaéorobe facultative. Toutes les restrictions concernant la disponibilité de cette souche seront irrévocablement levées lors de la délivrance d'un brevet correspondant à la présente demande ou une autre demande qui revendique le bénéfice de priorité sur cette demande.

Cette séquence de 9,2 kb comprend des gènes codant pour de nouvelle enzymes impliquées dans la synthèse de l'EPS ayant la structure répétée suivante.

β-D-Gal*p*      α-L-Rha*p*      β-D-Gal*p*
1      1      1
|      |      |
4      3      3
→3)-β-D-Gal*p*(1→4)-α-D-Gal*p*(1→2)-α-D-Gal*p*(1→3)-β-D-Glc*p*(1→

Grâce aux connaissances acquises sur la biosynthèse des EPS décrits dans la littérature, notamment de l'EPS décrit dans EP750043 (résultats non-publiés), on a pu ainsi identifier les nouvelles fonctions enzymatiques impliquées dans la biosynthèse de l'EPS ci-dessus. Les nouvelles enzymes impliquées dans la synthèse de cet EPS peuvent ainsi catalyser spécifiquement l'une des nouvelles liaisons suivantes entre sucres:
- la liaison osidique β-1,3 entre le carbone 1 d'un D-Gal*p* activé et le carbone 3 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique β-1,4 entre le carbone 1 d'un D-Gal*p* activé et le carbone 4 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique α-1,3 entre le carbone 1 d'un L-Rha*p* activé et le carbone 3 du sucre présent à l'extrémité non-réductrice d'une chaîne saccharidique en formation, ladite chaîne étant composée notamment d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1.

La chaîne saccharidique en formation peut être composée simplement d'une chaîne principale sans ramification de 1 à 4 D-Gal*p* et/ou D-Glc*p* liés entre-eux par des liaisons osidiques α-1,2, α-1,3, β-1,4 et/ou β-1-3.

A titre d'exemple, cette chaîne peut être choisie parmi les chaînes tetra-sacchridiques suivantes, ainsi que leurs précurseurs mono-, di-, ou tri-saccharidique(s):
α-D-Gal*p*(1→3)-β-D-Glc*p*(1→3)-β-D-Gal*p*(1→4)-α/β-D-Gal*p*-amorce;
α-D-Gal*p*(1→2)-α-D-Gal*p*(1→3)-β-D-Glc*p*(1→3)-β/α-D-Gal*p*-amorce;
β-D-Gal*p*(1→4)-α-D-Gal*p*(1→2)-α-D-Gal*p*(1→3)-β/α-D-Glc*p*-amorce ; et
β-D-Glc*p*(1→3)-β-D-Gal*p*(1→4)-α-D-Gal*p*(1→2)-β/α-D-Gal*p*-amorce.

La chaîne saccharidique en formation peut être aussi composée d'une chaîne principale comprennant en outre 1 ou 2 sucres ramifiés, tels que D-Gal*p* et/ou L-Rha*p*. Ces sucres ramifiés peuvent être liés sur la chaîne principale en β-1,3, β-1,4 ou α-1,3, par exemple.

A titre d'exemple, cette chaîne peut être choisie parmi les chaînes principales tetra-sacchridiques suivantes, ainsi que leurs précurseurs mono-, di-, ou tri-saccharidique(s):
α-D-Gal*p*(1→3)-β-D-Glc*p*(1→3)-[β-D-Gal*p*(1→4)]-β-D-Gal*p*(1→4)-[α-LRha*p*(1→3)]-α/β-D-Gal*p*-amorce;
α-D-Gal*p*(1→2)-[β-D-Gal*p*(1→3)]-α-D-Gal*p*(1→3)-β-D-Glc*p*(1→3)-[β-D-Gal*p*(1→4)]-β/α-D-Gal*p*-amorce;
β-D-Gal*p*(1→4)-[α-Rha*p*(1→3)]-α-D-Gal*p*(1→2)-[β-D-Gal*p*(1→3)]-α-D-Gal*p*(1→3)-β/α-D-Glc*p*-amorce; et
β-D-Glc*p*(1→3)-[β-D-Gal*p*(1→4)]-β-D-Gal*p*(1→4)-[α-LRha*p*(1→3)]-α-D-Gal*p*(1→2)-[β-D-Gal*p*(1→3)]-β/α-D-Gal*p*-amorce.

De préférence, une des nouvelles enzymes selon l'invention catalyse la liaison osidique α-1,3 entre le carbone 1 d'un L-Rha*p* activé et le carbone 3 du galactose présent à l'extrémité non-réductrice d'une des chaînes saccharidiques ci-dessus.

De préférence, une des nouvelles enzymes selon l'invention catalyse la liaison osidique β-1,3 entre le carbone 1 d'un D-Gal*p* activé et le carbone 3 du galactose présent à l'extrémité non-réductrice d'une des chaînes saccharidiques ci-dessus.

De préférence aussi, une des nouvelles enzymes selon l'invention catalyse la liaison osidique β-1,4 entre le carbone 1 d'un D-Gal*p* activé et le carbone 4 du galactose présent à l'extrémité non-réductrice d'une des chaînes saccharidiques ci-dessus.

Les nouvelles enzymes recombinées, identifiées à partir de la séquence d'ADN de 9,2 kb, ont l'une des séquences en acides aminés choisie dans le groupe de séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, et les séquences qui leur sont homologues.

La présente invention vise également tout ADN recombiné codant pour une enzyme selon l'invention, c'est à dire l'une des enzymes choisie dans le groupe de séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9 et SEQ ID NO:10. En particulier, cet ADN peut comprendre au moins un gène ayant l'une des séquences nucléotidiques choisie parmi les séquences délimitées dans la séquence SEQ ID NO:1 par les nucléotides 9-353, 516-1292, 1323-1982, 2074-3174, 3246-4373, 4511-5317. 5335-6204, 6709-7692, 7723-8784, et les séquences qui leur sont homologues.

La présente invention vise aussi tout vecteur recombinant comprenant un ADN selon l'invention. Ce vecteur recombinant peut être tout fragment d'ADN, simple ou double brin, linéaire ou circulaire, d'expression ou d'intégration, et comprenant une séquence d'ADN selon l'invention, notamment tout ou partie de la séquence SEQ ID NO:1. Dans le cas où le procédé décrit dans EP564966 ne serait pas utilisé (voir ci-après), il faut veiller à ce que le vecteur puisse exprimer l'ADN selon l'invention par des séquences nucléiques adaptées (promoteur; site d'attachement du ribosome; codon préféré par l'organisme hôte, etc.), et le cas échéant à ce qu'il comprenne une ou plusieurs origines de réplication cellulaire, notamment d'*Escherichia coli* et/ou d'un *Streptococcus*, par exemple.

Ainsi, pour opérer la biosynthèse d'un EPS, on peut intégrer tout ou partie de la séquence SEQ ID NO:1 comprenant au moins un des gènes précités dans une cellule hôte au moyen du procédé décrit dans EP564966, ledit procédé étant incorporé par référence dans l'enseignement de la présente invention. En résumé, ce procédé permet de pouvoir (1) transformer la cellule hôte avec un plasmide donneur qui ne s'y réplique pas, ledit plasmide comprenant ledit fragment placé fonctionnellement (le cadre de lecture est conservé) dans une partie d'un opéron issu de la cellule hôte; (2) identifier les transformants ayant intégré la totalité du plasmide; (3) sélectionner des transformants ayant uniquement intégré dans le chromosome le fragment selon l'invention, les autres séquences du plasmide s'étant excisées du chromosome; (4) et cultiver les transformants sélectionnés dans des conditions appropriées pour la production d'un EPS.

On peut noter que ce procédé permet de ne pas utiliser des séquences promotrice et d'activation traductionnelle fonctionnelles. De plus, les conditions de culture appropriées pour la production d'EPS sont à la portée de l'homme du métier, qui peut utiliser des milieux de culture standards, et choisir le pH, la température et l'agitation du milieu optimum selon la souche utilisée.

On peut aussi choisir de cloner tout ou partie de la séquence SEQ ID NO:1 comprenant au moins un des gènes précités dans un plasmide d'expression autoréplicatif en aval de séquences promotrice et d'activation traductionnelle fonctionnelles, et le cas échéant en amont d'un terminateur, puis de transformer une cellule hôte par le plasmide recombinant.

On peut aussi utiliser les enzymes recombinées pour modifier ou synthétiser *in vitro* un oligosaccharide ou un polysaccharide comme un EPS, par exemple. Pour cela, il est préférable de purifier au moins une de ces enzymes, en surexprimant classiquement leur gène dans une cellule, et en les isolant classiquement, par précipitation et/ou chromatographie (du milieu de culture et/ou de la partie membranaire des cellules), par exemple.

Un autre objet de la présente invention concerne une cellule comprenant, intégré dans son génome ou par le moyen d'un plasmide réplicable, un ADN recombiné selon l'invention, et exprimant une enzyme recombinée fonctionnelle selon l'invention. Cette cellule peut faire partie du groupe des moisissures, des levures, des bactéries et des plantes, par exemple. De préférence, les levures appartiennent aux genres *Saccharomyces, Kluyveromyces, Hansenula* et *Pichia* ; les bactéries sont Gram négative ou positive appartenant au genre *Escherichia*, *Bacillus*, *Lactobacillus*, *Lactococcus*, *Streptococcus* et *Staphylococcus ;* les cellules de plantes appartiennent aux légumineuses, et aux espèces céréalières et ligneuses ; tandis que les moisissures sont les cellules traditionnellement utilisées pour préparer un koji comme les *Aspergillus, Rhizopus* et/ou *Mucor*.

Un autre objet de la présente invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte; (2) on transforme une cellule hôte par ledit vecteur, ladite cellule hôte utilisant les enzymes codées par le vecteur, le cas échéant en combinaison avec d'autres enzymes produites par la cellule hôte si le vecteur n'apporte pas tous les enzymes nécessaires à la biosynthèse d'un EPS, pour la biosynthèse d'un EPS ; (3) puis on cultive la cellule hôte transformée dans des conditions appropriées pour la production d'un EPS.

Si l'on ne met pas en oeuvre le procédé décrit dans EP564966, le vecteur doit alors comprendre en outre au moins une séquence promotrice fonctionnelle et au moins une séquence d'activation traductionnelle fonctionnelle, permettant l'expression des gènes codant pour au moins l'une des enzymes selon l'invention.

La présente invention est décrite plus en détail ci-après à l'aide du complément de description qui va suivre, qui se réfère à des exemples d'obtention de fragments d'ADN, de plasmides recombinants et de bactéries transformées selon l'invention. Ces exemples sont précédés d'une description des milieux de culture. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook *et al*. (Molecular Cloning, A Laboratory Manual, Cold Spring Harbor Laboratory Press, U.S.A., 1989). Les pourcentages sont donnés en poids/poids, sauf indication contraire.

### Milieux: (rajouter 1,5% de Bacto-agar pour un milieu solide)

- M17 (Difco,USA): tryptone 0,5%, soytone 0,5%, viande hydrolysée 0,5%, extrait de levure 0,25%, acide ascorbique 0,05%, sulphate de magnésium 0,025%, disodium-beta-glycérophosphate 1,9% et de l'eau.
- LM17: milieu M17 comprenant 1% de lactose.
- GM17: milieu M17 comprenant 1% de glucose.
- MSK: lait écrémé (poudre reconstituée à 10%) comprenant 0,1% d'extrait de levure.
- LB: tryptone 0,5%, extrait de levure 0,25%, et NaCl 1%.

### Exemple I clonage de l'operon de L. bulgaricus CNCM I-800 impliqué dans la synthèse d'un EPS

I.1. Structure répétée de l'EPS: la structure de l'EPS produit par la souche *L. bulgaricus* Lfi5 (CNCM I-800) a été déterminée par résonnance magnétique, selon une technique similaire à celles décrites dans EP 97111379.0 et EP 97111381.6. Les résultats montrent que l'EPS est constitué de l'unité répétitive suivante: 
β-D-Galp          α-L-Rhap          β-D-Galp
1                 1                 1
|                 |                 |
4                 3                 3
→3)-β-D-Galp(1→4)-α-D-Galp(1→2)-α-D-Galp(1→3)-β-D-Glcp(1→
I.2. Clonage:
   L'approche tentée afin d'isoler les gènes *eps* de la souche Lfi5 est basée sur la comparaison de gènes *eps* connus (ceux de *S. thermophilus*) et de leurs homologues (gènes *cap, cps, rfb*) d'espèces variées, et même d'espèces non-apparentées phylogéniquement, comme des espèces Gram-négatif. Ces comparaisons permettent ainsi de définir des régions conservées entre les espèces, et d'utiliser des amorces nucléotidiques dérivées de ces régions conservées afin d'amplifier par PCR une partie interne de l'opéron *eps* de la souche Lfi5. Cette approche s'est malheureusement heurtée à une impossibilité d'amplifier un gène *eps*, quel que soit les amorces choisies. Les raisons ayant conduit à ces échecs peuvent être multiples, par exemple liées à une faible homologie entre les gènes *eps* de la souche Lfi5 et ceux des régions conservées, ou à des conditions de PCR inadéquates, etc.
   Face à ces échecs, on a décidé de sélectionner, puis d'utiliser dans une PCR, des amorces nucléotidiques particulièrement dégénérées. Le choix de ces amorces spécifiques a en fait été déterminant pour l'amplification d'un gène *eps* de la souche Lfi5, et a été motivé par les considérations suivantes: (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codés naturellement par 1 ou 2 codons, tel que la méthionine, le tryptophane, la glutamine ou l'acide glutamique, par exemple ; etc.
   Parmi les amorces testés, celles ayant les séquences nucléotidiques SEQ ID NO:11 et SEQ ID NO:12, ont permis d'amplifier un fragment de gène *eps* dans les conditions de PCR suivantes: successivement 2 min à 95°C, 1 min à 42°C, 20 s à 72°C, puis 35 fois de suite le cycle de 30 s à 94°C, 1 min à 42°C et 20 s à 72°C. Pour cela, l'ADN génomique de la souche Sfi39 a été au préalable isolée selon la technique de Slos *et al*. (Appl. Environ. Microbiol., **5**7, 1333-1339, 1991). On utilise environ 100 ng d'ADN génomique et la Taq polymérase.
   Un fragment de PCR de 143 pb a ainsi pu être isolé, puis cloné dans le plasmide linéarisé pGEMT (Promega, USA). Le séquencage de ce fragment par la méthode des didéoxynucléotides (kit f-mol® DNA Sequencing System, Promega) indique une séquence correspondant aux nucléotides 1698 à 1841 de la séquence SEQ ID NO:1.
   Ce fragment de PCR a été utilisé pour cribler une banque λ-ZAP Express (Stratagene, USA) renfermant des fragments d'ADN de la souche Lfi5. Pour cela, selon les recommandations du fournisseur on digère partiellement une préparation d'ADN dudit mutant par *Sau*3A, on sépare les fragments par une électrophorèse sur gel d'agarose, on coupe du gel les bandes correspondants à des fragments de 5 à 12kb, on élue l'ADN, puis on le ligue au vecteur λ-ZAP Express préalablement digéré par *Bam*HI. On encapside *in-vitro* le produit de ligation à l'aide du système GigagoldIII (Stratagene), on mélange ensuite les phages avec des *Escherichia coli* XL1Blue (Stratagene) selon les recommandations du fournisseur, puis on étale le mélange sur boîte de Pétri. On analyse ensuite les plaques recombinantes par hybridation de leur ADN transféré sur une membrane Hybond-N (Amersham Life Sciences, UK) avec le fragment de PCR préalablement rendu radioactif (kit Random Primed DNA Labeling, Boehringer-Manheim).
   Parmi les nombreuses plaques recombinantes, on a pu sélectionner par hybridation plusieurs plaques positives, desquelles on a ensuite isolé les vecteurs λ-ZAP Express, puis excisé les vecteurs pBK-pCMV (pCMV) renfermant l'insert chromosomique (voir les recommandations du fournisseur Stratagene). On a ensuite séquencé les inserts chromosomiques de ces vecteurs pCMV (kit f-mol® DNA Sequencing System). On a pu ainsi caractériser une séquence de 6 kb correspondant à l'opéron de la souche Lfi5 impliqué dans la synthèse d'un EPS.
   Afin de récupérer les séquences bordant le fragment initial de 6 kb, d'autres criblages de la banque Lambda Zap ont été effectués mais aucun n'a permis d'isoler de nouveaux fragments. Certaines séquences de *Lb. bulgaricus* contenant par exemple des promoteurs sont connues pour être toxiques et donc instables chez *E. coli*. Cela explique sûrement les difficultés à isoler les fragments d'ADN adjacents. Afin de contourner cet obstacle et de cloner les régions d'ADN bordant ce clone, les techniques de PCR réverse et de SSP-PCR (Single Specific Primer PCR) ont été utilisées. Cela a permis d'amplifier plusieurs fragments correspondant aux extrémités 5' et 3' de l'opéron *eps*. En recoupant les séquences nucléiques des différents fragments chromosomiques isolés, on a pu ainsi caractériser une séquence de 9,2 kb (voir la figure 1). La séquence nucléotidique de ce fragment de 9,2 kb est représentée dans la séquence SEQ ID NO:1 ci-après.
I.3. Analyse de la séquence SEQ ID NO: 1: la séquence SEQ ID NO:1 présente l'opéron *eps* de la souche Lfi5. Cette séquence comprend 9 ORFs complets, dans la même orientation, que l'on appelle *eps1, eps2, eps3, eps4, eps5, eps6, eps7, eps8* et *eps9* (voir la figure 1). La comparaison des séquences en acides aminés codées par les ORFs avec celles de protéines présentes dans la banque de donnée Swiss-Prot, à l'aide des logiciels FASTA, PEPPLOT et PILEUP de GCG-softwear, Wisconsin, USA, permet de déduire la fonction de ces protéines. Les résultats sont présentés ci-après.

L'ORF *eps1* (nucléotides 1323-1982) code pour une protéine (SEQ ID NO:2) ayant environ 45% d'identité avec la protéine Cps14E de *Streptococcus pneumaniae* (Genbank, n°P72513). Cet ORF code ainsi de toute evidence pour une galactosyl- ou glucosyl-phospho-transférase catalysant le transfert du premier sucre sur le transporteur lipidique ou protéique.

L'ORF *eps2* (nucléotides 2074-3174) code pour une protéine (SEQ ID NO:3) ayant environ 30% d'identité avec la protéine EpsF de *Streptococcus thermophilus* (Genbank, n°Q56043). Cet ORF code ainsi de toute evidence pour une alpha-glycosyltransférase.

L'ORF *eps3* (nucléotides 3246-4373) code pour une protéine (SEQ ID NO:4) ayant environ 34% d'identité avec la protéine EpsG de *Streptococcus thermophilus* (Genbank, n°Q56044). Cet ORF code ainsi de toute evidence pour une alpha-glycosyltransférase.

L'ORF *eps4* (nucléotides 4511-5317) code pour une protéine (SEQ ID NO:5) ayant environ 7% d'identité avec la protéine YveO de *Bacillus subtilis* (Genbank, n°P71054). Cet ORF code ainsi de toute evidence pour une beta-glycosyltransférase.

L'ORF *eps5* (nucléotides 5335-6204) code pour une protéine (SEQ ID NO:6) ayant environ 30% d'identité avec la protéine Cps14K de *S. pneumaniae* (Genbank, n°O07341). Cet ORF code ainsi de toute evidence pour une glycosyltransférase.

L'ORF *eps6* (nucléotides 6709-7692) code pour une protéine (SEQ ID NO:7) ayant environ 33% d'identité avec la protéine Eps I de *S. thermophilus* (GenBank n°Q56046). Cet ORF code ainsi de toute evidence pour une beta-glycosyltransférase.

L'ORF *eps7* (nucléotides 9-353) code pour une protéine (SEQ ID NO:8) ayant environ 38% d'identité avec la protéine EpsB *Lactococcus lactis* (GenBank n°O06030). Cet ORF code ainsi probablement pour une protéine de régulation impliquée dans le contrôle du poids moléculaire et/ou la longeur de la chaîne polysaccharidique.

L'ORF *eps8* (nucléotides 516-1292) code pour une protéine (SEQ ID NO:9) ayant environ 38% d'identité avec la protéine EpsC *Lactococcus lactis* (GenBank n°O06031). Cette homologie confirme le rôle de cet ORF dans la synthèse d'un EPS.

L'ORF *eps9* (nucléotides 7723-8784) code pour une protéine (SEQ ID NO:10) ayant environ 24% d'identité avec la protéine YveQ *Bacillus subtilus* (GenBank n°P71056). Cet ORF code ainsi probablement pour une protéine reponsable de la polymérisation des unités répétées.

En conclusion, les homologies observées indiquent que les différents ORFs identifiés dans les inserts chromosomiques sont impliqués dans la biosynthèse de l'EPS.

### Exemple II Biosynthèse d'un EPS

On prépare un plasmide à partir du plasmide pSA3 (Dao *et al*., Appl. Environ. Microbiol., 49, 115-119, 1985), en ajoutant un fragment d'ADN de la souche Lfi5 contenant la séquence de 9,2 kb décrite à l'exemple I (voir la liste de séquence), et en ajoutant les parties régulatrices de l'opéron *eps* de la souche *S*. *thermophilus* Sfi6 (EP750043), de sorte que l'expression (la transcription et la traduction) des gènes *eps* de la souche Lfi5 puisse être effectuée dans *S*. *thermophilus*. On transforme ensuite par électroporation, avec ce plasmide, la souche *S. thermophilus* CNCM I-1422 qui a été déposée le 18 mai 1994 selon le traité de Budapest. N'importe qu'elle autre souche filante *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal=2:2.

### Exemple III Biosynthèse d'un EPS

On prépare un plasmide à partir du plasmide pSA3, en ajoutant un fragment d'ADN de la souche Lfi5 contenant la séquence de 9,4 kb décrite à l'exemple I (voir la liste de séquence), et en ajoutant les parties régulatrices de l'opéron *eps* de la souche *S. thermophilus* Sfi6 (EP750043), de sorte que l'expression (la transcription et la traduction) des gènes *eps* de la souche Lfi5 puisse être effectuée dans *S. thermophilus*. On transforme ensuite par électroporation, avec ce plasmide, la souche *S thermophilus* CNCM I-1351 qui a été déposée le 5 août 1993 selon le traité de Budapest. N'importe qu'elle autre souche filante *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal:Rha=1:3:2.

### Exemple IV Biosynthèse d'un EPS

On isole de l'ADN chromosomique de la souche CNCM I-800, on digère la préparation d'ADN par *Xba*I, on sépare les fragments d'ADN par électrophorèse sur gel d'agarose 0,7%, on élue les fragments de plus de 9 kb, on ligue l'ADN extrait au vecteur pJIM2279 (obtenu de P. Renault, INRA, Jouy-en-Josas, Paris, France) préalablement digéré par *Xba*I puis déphosphorylé. On transforme la souche *Lactococcus lactis* MG1363 (J. Bacteriol., 154, 1-9, 1983), cultivée sur milieu GM17 à 30°C, par la méthode de De Vos *et al*. (Gene, 85, 169-176, 1989). On sélectionne les clones transformés par hybridation de l'DNA génomique des clones avec l'une des sondes dérivées de la séquence SEQ ID NO:1

### Exemple V Préparation de gènes fonctionnels homologues aux gènes eps1-9

On prépare des dérivés fonctionnels des gènes *eps1-9* en mettant en oeuvre une méthode adaptée de celle décrite par Adams *et al*. (EP402450; Genencor). Pour cela, on isole chacun des gènes *eps* par PCR à partir de l'ADN génomique de la souche Lfi5 (CNCM I-800). Les amorces utilisées sont choisies à partir de la séquence SEQ ID NO:1, de sorte à amplifier uniquement chaque gène. On clone chaque produit de PCR dans le vecteur d'expression pQE60 (Qiagen, US) fonctionnellement en aval d'un promoteur *E. coli* régulant la transcription et la traduction du gène *eps*. On soumet enfin chaque vecteur à une mutagénèse chimique *in-vitro* avec de l'hydroxylamine, comme décrit par Adams *et al*..

On transforme la souche *E. coli* DH5α (Stratagen, US) qui ne possêde pas naturellement d'activité glycosyltransférase, par les plasmides pQE60 traités avec l'agent mutagène, puis on sélectionne des transformants.

Pour identifier parmi les transformants des gènes *eps* codant pour des variants d'enzymes, on analyse la spécificité enzymatique des enzymes exprimées dans chaque clone d'*E.coli*, au moyen de la méthode décrite à l'exemple VI. L'analyse des gènes *eps* portés sur les plasmides pQE60 des clones ainsi sélectionnés montre que ceux-ci ne différent du gène *eps* original que par la substitution, la délétion ou l'addition d'un petit nombre de bases nucléotidiques conduisant à des modifications de la séquence en acides aminés de la glycosyltransférase.

### Exemple VI Synthèse de polysaccharides avec les glycosyltransférases de Lfi5

On isole chacun des gènes *eps* codant pour une glycosyltransférase par PCR à partir de l'ADN génomique de la souche Lfi5 (CNCM I-800). Les amorces utilisées sont choisies à partir de la séquence SEQ ID NO:1, de sorte à amplifier uniquement chaque gène. On clone chaque produit de PCR dans un vecteur d'expression pQE60 (Qiagen, US). Les vecteurs ainsi traités sont ensuite introduits dans la souche *E. coli* DH5α. On cultive les souches DH5α transformées dans du milieu LB contenant de l'ampicilline jusqu'à une DO₈₀₀ de 0,8, on ajoute au milieu de culture 1 mM d'IPTG, on incube les cellules pendant 2 h, on centrifuge les cellules à 6000 g pendant 10 min à 4°C, on les lave dans le tampon A (50 mM de Tris-acétate pH8, 1mM de DTT, 1 mM d'EDTA et 10% de glycérol), on les centrifuge à nouveau, on re-suspend les cellules dans le tampon A contenant en outre 1 mM de PMSF (fluorure de phénylméthane sulfonyle) et on soumet la suspension à 15000 psi. On élimine les cellules entières par centrifugation à 6000 g pendant 15 min à 4°C, on récupère les membranes cellulaires du surnageant par ultracentrifugation à 100000 g pendant 1 h, et re-suspend l'aliquot dans le tampon A. Ce protocole permet ainsi généralement d'obtenir environ 10 mg/ml de protéines.

Parallèlement, on cultive la souche Lfi5 dans un milieu contenant du D-Glc*p*, D-Gal*p* et L-Rha*p*, on soumet les cellules de cette culture à 20000 psi, on extrait des membranes les chaînes saccharidiques en formation (liées à une amorce) en traitant les débris cellulaires avec du chloroforme/méthanol (2:1) et en évaporant l'extrait. A titre de témoin dans les essais qui vont suivre, on procède de la même manière avec la souche Lfi5 cultivée en présence de sucres radioactifs.

On détermine ensuite la spécificité des glycosyltransférases présentes dans les membranes par un procédé similaire à celui décrit par Kolkman *et al*. (Mol. Microbiol., 26, 197-208, 1997). Pour cela, on fait réagir pendant 2 h, dans un volume réactionnel de 150 µl, 100 µl (environ 1 mg) de membranes, 50 mM de Tris-acétate pH8, 10 mM de MgCl₂, 1mM d'EDTA, 1 µl (environ 25 nCi) d'UDP^{[14C]}-Glc*p*, d'UDP^{[14C]}-Gal*p* ou de TDP^{[14C]}-Rha*p* selon la glycosyltransférase considérée, et le dépôt contenant les chaînes saccharidiques en formation non-marquées isolées de la souche Lfi5 (voir le paragraphe ci-dessus). Pour arrêter la réaction, on ajoute au volume réactionnel 2 ml de chloroforme:méthanol (2:1), on agite vigoureusement le mélange, et on le laisse reposer pendant 30 min. On élimine les sucres nucléotidiques en extrayant la phase organique 3 fois de suite dans 0,4 ml de PSUP (pour 1 litre: 15 ml de chloroforme, 250 ml de méthanol, 1,83 g de KCl et 235 ml d'eau). La phase inférieure contenant toutes les chaînes saccharidiques liées à une amorce est ensuite séchée sous vide, resuspendue dans 200 µl de 1-butanol et séparée dans deux tubes.

Dans la première série de tubes, on détache les oligosaccharides de leur amorce en effectuant une hydrolyse douce dans 50 mM de TFA (ac. trifluoroacétique) à 90°C pendant 20 min.

Dans la deuxième série de tubes, on soumet les oligosaccharides à une hydrolyse poussée dans une solution de 4M TFA pendant 1 h à 125°C

On sèche ensuite les hydrolysats et on les resuspend dans 5 ml de sucres transporteurs dans 40% d'isopropanol (5 mg/ml de chaque sucre suivant: Glc, Gal, GalN, maltose, maltotriose, maltotetraose). Les sucres hydrolysées de chaque série de tubes sont alors déposés sur une plaque HPTLC silicagel 60 (Merck) que l'on soumet à un éluant composé de chloroforme, d'acide acétique et d'eau (respectivement 6:7:1). On autoradiographie ensuite la plaque pendant 16 à 24 h avec un film Biomax MS (Kodak). On visualise les sucres transporteurs en diffusant sur la plaque une solution d'éthanol comprenant 5% d'H₂SO₄, et en chauffant la plaque à 100°C pendant 15 min. L'analyse des plaques permet ensuite l'identification précise des fonctions enzymatiques codées par les gènes *eps* de la souche Lfi5.

LISTE DE SEQUENCES

(1) INFORMATIONS GENERALES:
    (i) DEPOSANT:
        (A) NOM: SOCIETE DES PRODUITS NESTLE
        (B) RUE: 55 AVENUE NESTLE
        (C) VILLE: VEVEY
        (D) ETAT OU PROVINCE: VAUD
        (E) PAYS: SUISSE
        (F) CODE POSTAL: 1500
   (ii) TITRE DE L' INVENTION: BACTERIES LACTIQUES PRODUISANT DES
            EXOPOLYSACCHARIDES
  (iii) NOMBRE DE SEQUENCES: 12
   (iv) FORME DECHIFFRABLE PAR ORDINATEUR:
        (A) TYPE DE SUPPORT: Floppy disk
        (B) ORDINATEUR: IBM PC compatible
        (C) SYSTEME D' EXPLOITATION: PC-DOS/MS-DOS
        (D) LOGICIEL: PatentIn Release #1.0, Version #1.30 (OEB)

(2) INFORMATIONS POUR LA SEQ ID NO: 1:
    (i) CARACTERISTIQUES DE LA SEQUENCE:
        (A) LONGUEUR: 9217 paires de bases
        (B) TYPE: nucléotide
        (C) NOMBRE DE BRINS: double
        (D) CONFIGURATION: linéaire
   (ii) TYPE DE MOLECULE: ADN (génomique)
   (ix) CARACTERISTIQUE:
        (A) NOM/CLE: CDS
        (B) EMPLACEMENT:1323..1982
        (D) AUTRES INFORMATIONS:/product= "esp1"
   (ix) CARACTERISTIQUE:
        (A) NOM/CLE: CDS
        (B) EMPLACEMENT:2074..3174
        (D) AUTRES INFORMATIONS:/product= "eps2"
   (ix) CARACTERISTIQUE:
        (A) NOM/CLE: CDS
        (B) EMPLACEMENT:3246..4373
        (D) AUTRES INFORMATIONS:/product= "eps3"
   (ix) CARACTERISTIQUE:
        (A) NOM/CLE: CDS
        (B) EMPLACEMENT:4511..5317
        (D) AUTRES INFORMATIONS:/product= "eps4"
   (ix) CARACTERISTIQUE:
        (A) NOM/CLE: CDS
        (B) EMPLACEMENT:5335..6204
        (D) AUTRES INFORMATIONS:/product= "eps5"
   (ix) CARACTERISTIQUE:
        (A) NOM/CLE: CDS
        (B) EMPLACEMENT:6709..7692
        (D) AUTRES INFORMATIONS:/product= "eps6"
   (ix) CARACTERISTIQUE:
        (A) NOM/CLE: CDS
        (B) EMPLACEMENT:9..353
        (D) AUTRES INFORMATIONS:/product= "eps7"
   (ix) CARACTERISTIQUE:
        (A) NOM/CLE: CDS
        (B) EMPLACEMENT:516..1292
        (D) AUTRES INFORMATIONS:/product= "eps8"
   (ix) CARACTERISTIQUE:
        (A) NOM/CLE: CDS
        (B) EMPLACEMENT:7723..8784
        (D) AUTRES INFORMATIONS:/product= "eps9"

(xi) DESCRIPTION DE LA SEQUENCE: SEQ ID NO: 1:

GGCCAACTAT GCACTCTAYT TTCAATGTAT CCAANNGCAA CSGCTTGACC ACTTTGTTGA 60
CTAGCCGGTC TATGGAAATG GACGCAAACA GCGTGATCCG GGAAAGTGGT GTAGAGAACC 120
TGTCAATCTT GACGGCAGGT CCAATTCCGC CAAACCCATC AGAACTTCTG TCTTCCAAGC 180
ATATGTTGGA TTTGATTGAA GATTTAAAGC AAGAATATGA TATGGTCGTG CTTGACTTAG 240
CACCGATCTT GGACGCGGGC GAAACCCAGC AACTGACCAG TTCTTTGGAC GGGACGATCT 300
TGGTTGTGCG CCAGTCATAT TCACAGAAGT CAGCGGTTAA GCGGGGCAGT TGAGCTACTT 360
AAGCTGACTA AGTCACCAAT TTTAGGTTAT GTTATGAACG ATGTTGATGC CGATGGGGAT 420
GAGACGGCTA TGGATACGGG TABGGCTATG GTTACGGCTA CGGATATGGC GAAGAAGAGG 480
AGAAGAAGGG GCTCTTTGGG AGAAAGAAGT AGCTCATGCC GGTAGTTGAT TTACATTGTC 540
ACATCTTACC AGGAATCGAC GATGGTTCAA AGTCTTGGGA GGCTTCACTT AAGCTCGCTA 600
GAGCCGCGGT TGCGGATGGC GTAACCCATG CCTTGTGTAC TCCACACACA TTAAATGGCC 660
GCTACACCAA CCACAAGCAA GATGTGATCA AGTTGACGGA AGAGTTTCAA GACCGGATTG 720
ACCAAGCGGG TATTCCGCTG ACGGTCTTTC CAGGACATGA AGTCAGATTG TCAGGTGGCT 780
TAACTGAAGC TCTTGACAAT GATGACATCC TCTTCTGCGA TGAAGAGGGA CACTACATGC 840
TCTTGGAATT GCCAAGCAAT GAAGTGCCAC ATTACACCAA GAACATGGTG TACGAACTGA 900
CCAGAAGAGG GATTACGCCA ATCGTTGTTC ACCCAGAACG GAATAAAAAA ATTCTGGCAA 960
ATCCGCAGAA GTTGCAAGAA TTCTTGGAAA TGGGAGTTTT GGTACAGATC ACTGCCAGCT 1020
CATATACCGG CTTGTTTGGC AAAGAAATCG AAGACTGCAG CCGGGAGTTT ATCAAGGCTG 1080
GACAATGTGC CTTCTTTGCC AGTGATGCTC ATGATTTGCC AAGACGGCAA TATCAACTGA 1140
GTGAAGCACT GGATAAGTTG GCGAAAGAAT TTGGTGAAGA TAAGAAACAG AAGTATTTAG 1200
ATAACGCGCA AGCGGTAATC AATGGCGATC CGGTTTACTT GAACTGGCAA CCTTTACCGA 1260
AGAAGCGCAA GAAGTTCTTA GGTCTGTTTT AAAGTGGAAA TTTGGATGAA GGGGAGGAAA 1320
AAATGGATAT GGGATATAGC AGTAGTCCAG CATATGTTAA ACAGTCCAAA GTCAAGGGCC 1380
GTCCTCTCTA CCATGGGATC AAGCGTCTCT TTGACTTTCT GGCTTCTTCA ATTGCCTTGA 1440
TTCTATTGTC GCCACTTTTT TTGTGGCTGG CAATCAAGAT TCATGGTGAA GACGGCGGAC 1500
CAGTCTTTTA CTCACAAATC AGAATTGGCA AGGATGAAAA GCCTTTCCGG ATCTACAAGT 1560
TTCGGTCCAT GGTGGTCAAT GCTGAGAAGC TGAAGAAGGA TCTGCTGGAG CAAAATGAAG 1620
TCGAAGGGGC CATGTTTAAG ATGCATGATG ACCCGCGGAT CACTAAGATT GGCAAGTTCA 1680
TCAGAGCACA CAGCTTGGAT GAATTGCCTC AGCTTTGGAA TGTGCTGATT GGGAATATGT 1740
CACTGGTGGG GCCTCGGCCA CCGTTGCCAA ATGAGGTTGA ACAGTATACT GACTATGACA 1800
AGCAGAGATT GCTCGTAAAA CCAGGCTGCA GTGGCCTTTG GCAAGCAACT AGCCGGAATG 1860
CGGCTGATTT TGCGGATATG GTGCAGCTCG ACATCGAGTA CATTAATCGG AGTGGCGTCT 1920
TCTTTGATCT GTGGATCATT TTCAAGACAA TCGGTGTTAT GTTCCACCCG AACGGGATGT 1980
AGATGTATGA GGAGAATGAC GAATAAGCTA TATGGAAAAG TGGCTAAGCA CTTGTTGTTC 2040
TTCAAGAGCG AATGATTATA GGGTTAGGTA CATATGAAAG GAAATAATAA ACCTCGCCTG 2100
CTCTACTTCG TCGAAGCGAT GGGCGGCGGG GTTTTTACAT ATATTGTTGA TCTAGCAAAT 2160
AGCCTAGTGG ATGATTGGGA TGTTTATATT GGTTATGCTA TGCGCAACCA AACACCTCAA 2220
AATTATCGAG AGTATTTCGA TGAACGAGTG CATCTAATCG AGGTTAAGAA TTTTGCTCGT 2280
AGTACTAGCA TCACGAAAGC AATCAAAGCA GGACAAGAGA TGAAAAGGAT AGCAAAGGCT 2340
ATTCGGCCAG ATGTTATTCA TCTACATTCT AGTATTGCAG GAGCAATTGG ACGTGTCGTC 2400
TTTAACACAA AGAAAACGCC GGTCTTTTAT ACTCCACATG GTTACAGCTT CTTAATGCAA 2460
GGCGAGAGTA GCAAGAAACG CTTGGCGTAT AAGTTGGTAG AGCAGTTTTG CGGCAAGAGC 2520
CAAGCAACGA CCATTGCATG TAGCCCTGGT GAGTATCAAG AAGCTTTAAA AGTCTCTAAA 2580
CATGCCGTGG AAGTTGATAA TGGTATCAAC ATTGAACAAT TGCAAGAATT GATAGACACG 2640
ACGGACGCTT CGAAGATTGA TCATTATGAC GTTTTTACAC TGGGGCGTAT CTCAGTACAG 2700
AAGAATCCAA ATGTTTTCAA TGAGGTCGCA TTGAAGTTGT CGAATTTAAA ATTTTTGTGG 2760
ATTGGCGATG GTGAATTACG TTCAGAGCTA ACTGCTCCGA ATATTACTGT TACCGGTTGG 2820
TTAACGCGTC ATGAGGCGTT AAAGTACTCA CTTAATAGTG ATACTTTTAT GCTTACCTCA 2880
CTGTGGGAAG GGTTACCGAT GAGCTTGTTG GAAGCAATGT ACATGAAGAA ATTATGTGTG 2940
GTCAGTGATG TCATCGGTAA CCATGATGTA ATCAATGATG GTGTTAATGG GTATGTATGT 3000
CAGACCGTTA ATGATTTTTA TAATAGAATA TCAATGATCG GGGGGGCTCG CTATTCTCTG 3060
ATTGATTGTG CTTATAAAGA TGTTTTAGAA CGCTACAATA CTAAAGTAGT GTCTAAAGCC 3120
TATGATCAAC TTTATCAAGC AGCAATCAAG AATAGTGAGT GTTTCTGTCT ATAAACTTTA 3180
AATTTTACTG AATAGTATCT GCTTGATTAA GTTATCTTCC AATCAATCTC TGCGAGAAAA 3240
ATCTTATGCA GAAAATACGT GTTGTTCATG TGGCTGAAGC TGCAGGTGGA GTTGAAAGAT 3300
ACTTGTATGC ACTGTTGAAG AACAGTGATT CTCAGAAGAT AGAGAACTAC CTTATAGCTT 3360
CCCAGCACTA TGAGATGGGG AAGTTTAAAA AATATGTTTC TGGTGAATAC CAACTTCAAA 3420
TGGCTCACGC AGCTTGTTTC AAAAATGATA AAGCTACAGT TAAGCAGATT AAAAAAACAG 3480
TTAGACAGAT CAAGCCAGAT ATCGTCTATG CACATTCAAC TAAAGCTGGA GCGCTTACTA 3540
GAATTGCTTT GATGGGGATG CATATACCAG TAATCTATAA TCCCCATGGT TGGGCATTCA 3600
ACATGGCTCA GTCCAAGAAG AAAGAACTGG CTTACAGGCT GATTGAGAAG GTTCAAATTC 3660
CCTTTACTAA AAAAGTTGTC TGTATTTCGG AAGCAGAGAT GCAGTCGGCG ATGCAAAAAC 3720
ATATTTGCTC AAAGGATAAA ATCAAAATTA TTCCCAATGG TATTGATTTT GATTTATTGG 3780
ATAAGGTGCA ACCTGTTACT CGGAGAGATC TTGGTATTCT CGAAGACGCA TTTGTTGTGG 3840
GACAAATTGG GCGCCTTAGT GAGCAGAAAT CCCCAGATGT CTTTGTTGAA ATGGCGGAGA 3900

AAATCAAGAA AGAAATACCA AATTCGTTCT TCGTGATGGT TGGTGATGGC AACCTTGAAG 3960
CTGAGATTCG TAGATTAATT AAATCGAAAG GTTTAGAGGA TTCTTTCTTG ATCACTGGAT 4020
GGGTTGATAA CCCGACCGGT TATTTAAATT GTTTCGATGT ATCAACGCTT CTGTCGAGAT 4080
GGGAAGGCTT TGGCTTGGCC TTAGTCGAAT ATATGTATTG CGGTGTGCCA TTAGTTTCAA 4140
CCAAGGTTGA TGCTATCCCA TACGTAGTTG ATGATGGCGT TGACGGGTTA TTGGTTGAAC 4200
CCGATAACCC ATATGAGGCG GCTAAGGCTG TAATCCGCAT TCACGAAGAT CCTGCTTTAG 4260
CAGCCAAATT TGTGGCTAAT GGTTTGAGTA TTGCGAAAGA TAAGTACGAC GTTCGCCGAG 4320
TCGCCAAACA AACACTGAAA TTATACTATA AAGTTTTGCA CAACTTAAAA TGAAGTGCAT 4380
AATTTGCGTT GTATATTTAT ACGTAACCGA TAATGCGTTA AATACAAATG GTGTTGGGAA 4440
AGCATCTGAA AGTTGTATAG TTTATAGTGT TGAATAGCTA AATTAAAATT AGATAATTAG 4500
GAGCAAGCCT ATGCCAAAAG TTTCAGTGAT TATGTCTATA TATAATTGCA AGAACTATAA 4560
AGCCTTAGAG AAGAGTATTG AATCAATTAT TGATCAGACC TACTCTGACT GGGAGTTTAT 4620
CATCTATAAT GATGGGTCGG AAGACGATGG TAAGACCTCA GAGTATCTCA AGAAATTGGG 4680
TAAACGTGAT TCAAGGATCC AAATCATTGA TTGTCCGAAT AATCATGGTT TGGCATATGC 4740
AAAGAATGAA ATGATCAAGG TCGCAAGAGG AGATTATATC ACAGCTCAGG ATGATGATGA 4800
TGTTTCGCAT CCTATGCGTT TGGCCCGAGA AGTAGACTTC TTAAATAGGC ACCCAGAGTA 4860
TGCCTTTGTT GGTACTGTTG CGAAAGTTTT TGATAATAGT GGTACGTGGG GACACTATGG 4920
ATTGAAAGAG AAACCAACTA AGAATACTTT TTTATGGAAT AGTCCATTTC TTCACCCATC 4980
TGTGATGTTT AGACATGAAG TGTTAGATCA AGTTAATGGG TACCGTGTTG CCAAGGATAC 5040
TATGCGTGCA GAAGATTATG ATCTATTCTT CAGACTGTAT GCTAAAGGTT ACAAAGGATA 5100
TAATATTCAG GAGGATTTAT ACGAATATAG GATTGAAAAT AATCCAAATA AGAAATATAG 5160
GCCGATGAGT GACCGTATTC AAGAAGCAAA GGTTCGATAT AAGGGGTTTA AGTCTTTTGG 5220
CATGTTAACG AAGGGGATTC CGTATATTAT TAAGCCCATC TTAATTGGAT TAATTCCGCA 5280
GAAGATATTT TATATAATTC GTAAGAATCG TTACTAGTGT GAGGGTAACC TATAATGTAC 5340
AAGAATGATT TATCTTTTGT CATGTCAAAG ATATTGGGGA GACTACCGGC TAAAAGAATT 5400
AGTGATAAAT TTAATATCTA TCGTCATCAA CAAATTAGGA AGCAATTAAC TCCTGTTTTT 5460
GACGAGGCGT ATGAAACATT TACGAGATAT GATAGCCATA AAGTAAACGA TACGATATGG 5520
ATTTTTTGGT GGCAAGGTGA AGATAGTATG CCTAAGGTAG TGAAAAAATG CTATCGCTCT 5580
ATCCAACAGA ATCGAGGTAA AAAAAATATT GTTTTGATCA CTCAAGACAA TATTAAACAA 5640
TATACAGACA TCCCTAAATT TATTTACGAC AAATTGGATA AAGGAGTAAT CACTTATACC 5700
CATTTTTCGG ATATTGTGAG GGCGAATTTA ATCAAAAATA ATGGTGGACT TTGGATGGAT 5760
GCTACGCTGT ATGTTACCTC TTCGCTAGAC AATATTGACC TAAAAAAATT ATTTTATTGT 5820
AGTGGCTATC CTGCTGATAC CTTTAATGTT AGTTTCGGAC GTTGGACGGG GTTTTTTATG 5880
GGCGGCCCTA GCGGAATGGA CCTTTTTTCT TTTATGGATC GTCTATATGA AGTATATTGG 5940
CGAGATCACG AAAAGCTGTT TGATTATTTC TTGATAGATT ATGGATTGGA TTATGCTTGG 6000
AAAAAAGATT TAAGTTCGTT TAAATCGCTA GAAGAGAGTT ATAAGCAAAA TAATCCTCAT 6060
CTGTTCGATT TGCAAGGAAT GTTAAACCAA CCGTATGATG ATAAAGAGTG GAAAAGAATA 6120
ACTAGCGATA CAAGCGTATT TAAACTGTCA TACAAGAAAA AGGTAGATTT TGATAAAAAG 6180
GAAAGCTATT ACAATAGATT GTGAAAATGA AAGACATGAC TAAAAAAATT CTATTGCAAT 6240
GACGACCTAC AACGGAAGTC AGTACATTGT AGAGTTATTA GATTCTCTTC GGCAACAGAC 6300
ACGTCCTGTT CAAGAAGTTA TTGTCTTACC TTCCATTTGG AATGATCCAG CACTGTTAGC 6360
TGTGACTGTT AGCTGTGATT GAAAGTATTA CAGCAGGCAA GCCATTGATT ACTACGTATT 6420
CAGGTGGGAT TCCTGAGTAT GCCGATAAGA AAGATGCGAT TATCTTGCCG ATAAATGACC 6480
AGTTAGTTGA GAATTTAGTT AATTCGTTAA CCAAATTAGC TAACGACAAG AAATTGCGTA 6540
TTAGTCTTGA ATAAGCAGCT AAAAAAAGAA GTCAGACATG GACTAAGGAA AGTTTTTATG 6600
ATGACTTTGT AGAAATAGTA GGTTAGTAAG GTGATACGCA GTTATGATCC TAAATCTAAG 6660
TATGCGTCAG CAGTAAAAAG TCAAGTTTGT AGGAAAGGAA TCATTGTAAT GAAGTTAAGC 6720
ATTATTGTCC CTGTTTATAA AGTGGAAGAA TATTTGAAAG ACTGTGTAAA TTCGATTTTA 6780
AATCAGACTT TTCATGATTT CGAGCTCATT CTTGTAGATG ACGGTTCACC AGATTCATGT 6840
CCTAAAATTT GTGATGAATA TTCTCAAAAG TTTAATAACG TTAAAGTAGT TCACAAACAA 6900
AATGGCGGAT TGTCTAGTGC TAGAAATGCT GGTATGAAAG TAGCAACTGG AGAGTATATT 6960
TCTTTTATTG ATAGTGATGA TTATCTTGCC TCAAACATGT ATGAGCATGT ATTTTCGATT 7020
ATGAAAAAAG AATGTGCTGA TATTGTTGTG GTGGGCAGAT GCTATGTGTA CCCAAATGGT 7080
TCCAAAAAAT TAAGAGAGAA ACAGAATGTG TATGAAGTTA TGGATGGCCC TAAAGCAACG 7140
GCCATAATGA ATACATCTTT GTTAGGGTAC TTTGATGCTG CAGCTTGGGA TAAAGTATAT 7200
AAACGAAGCT TGTTTAATGA CGTGAGTTAT CCCGAGGGGA AACTAAGTGA AGATTGGTAT 7260
ACTACATATA AAGTGTTTGC AAAAGCTAAT AGAATAGTTT ATGATTCAAC ACCCATGTAC 7320
TATTACAGAC AACGTGGAGG AAGTATTACC CATACAAGTT CTACAGTTAA TTATGACGCA 7380
ATGTATGCTA GTCGAGAAGT CTATGATTTC GTAAAAAAGA GACAACCGGA GTATACGGCT 7440
GAAGCAAATT TTGCATATGT TTTTTCAAGA ATTGGAGTTA TAGACAATTT AACTGTTCAG 7500
CCTACTGTTG ATAAACAAAA AATTAGGAAA ATACGTAATG ATATGAAGGC AAATATTAAA 7560
CAGTTAAAAA AGACAGACTG TTTTTCTAAG TTGAGTAAAA AAAGAAAAAT ACAATTGTTT 7620
TTAATTGAGC ATTGCTTAAA CTCATATACA ATTATTTTTA GGCGTGTAAA AAGCCTTGGC 7680
ATATCAGATT AAAAATAACA ATAACTACAA AGGATAAAGA ATATGTCTGT ATATTTTGGG 7740
CTGCTAATTT TTTGTGTAAT ATCCTCAATG ATCGGGCGCG GACTAACAAT AAATAGATTT 7800
GACATTAGAG CAAAGATAGT AGATGTATTA CCTTTTTTAG CTTTGTTTGT TGTATCTGCA 7860
TTTAGGTATC AAGTTGGTAA GGACTACGGT ATATATGTCG GTACGTATGA TTTAATTAAA 7920
ACAGGACAGC AAGCGAGAAT CGATATCTTC ACTTATTGGG TTATGAAGGT AATGGCTAGC 7980

TTGAATCTTG ACGTTCAGTA CTTTTTTATT GTTTCTAGTT TTATTGTAAA TTATCTAATC 8040
TACTTAGCTG TTCGAGATCA ATCAAAAGAT AGAACACTTA GTTATTATAT ATATATATGT 8100
GGGACTTTAT ATTTTGCCTC AATGAACACA GTTCGCCAGA TGATGAGTGT TGCTATGTTT 8160
TACTTTTCAT TGAAATATGT CGAAAGCAAT AATTGGAAAA AATATTTTTT ATGTAATTTG 8220
ATAGGTGGAC TTTTTCACCA AACAGCTTTT ATATTCCTGC CCATATATTT TGTTTTAAAG 8280
AGATATATGG GGTGGAAGAA ATATTTAGTA GTAATTATTA TAGCTATAGC ACTCAAGAAC 8340
CCAATCGTTA ATCTGCTTAA TGGGCTTATC TTAAATTCAA AATATGCGAT GTATGTCACT 8400
TATTCCAACT TTGCAAATAC AGCGTGGGGA GGATGGAAGA TAAGCAATTT CATTAATTTA 8460
ATATTATTTT TAGCATACTG CCTTTTGCTT AAAAATAAGG ATACTGAAGA TAGTATATAT 8520
ACTAATATTC ATTTTGTTGG GGTACTATGT TCGATTTTAC TACTTGGTAT CCCGGGTGGT 8580
GACAGAATTT TTCTTGGGTT TAGATTTATC GAATTATTAA GTGTACCCAA CTTACTCTAT 8640
AAATTAAAGT TAAGAAAAAT AAGCTATATT ATTTTGAGTT TAGTAATTGC AGTACTATAC 8700
TTGTTTTACT TTTGCTATAC GATCGGCGTA CAAAATGGTA ATTCTGTACT GCCATACGTG 8760
ACAACCCTAT TTTCAATAAA CTAATAGTAA TTTGATAGGA GTAGAACGTT GAAGTTTTTA 8820
ATAATTGATG ATGAACGTAG CCTCTATAAA GCTATGTTCA GTGATTTGAT AAATAGCTCG 8880
GATAAAAATA TTTCTGAGGT ACCTATATTC AGTCAAATGC CAAGATATCT ATCGGTTGCT 8940
AAGCCAATGG TATTTAATGA TCGGATTAAT CGACATGTGT GGCTACCGTT CAAAAAAACA 9000
TATGATAAAT ACTATTCTCT CACATCATAT AACTTTGATA AAAGCGAAAA ATACTGGGTA 9060
GTGATGCTAA ACGGAACATT AAATACACAC TATTCCGCTG AATATATAGC ACGATTAAAA 9120
AAGGAAAAAC CGAATGTCAA GTTTGCACTT ATCATGTACG ATCATTTCGG AAATCGAGCT 9180
GCAAGAAGAG TTAAAAAACT TTTACCGTTT TTTGATC 9217

(2) INFORMATIONS POUR LA SEQ ID NO: 2:
(i) CARACTERISTIQUES DE LA SEQUENCE:
(A) LONGUEUR: 219 acides aminés
(B) TYPE: acide aminé
(C) NOMBRE DE BRINS: simple
(D) CONFIGURATION: linéaire
(ii) TYPE DE MOLECULE: protéine
(xi) DESCRIPTION DE LA SEQUENCE: SEQ ID NO: 2:

Met Asp Met Gly Tyr Ser Ser Ser Pro Ala Tyr Val Lys Gln Ser Lys
1               5                   10                  15
Val Lys Gly Arg Pro Leu Tyr His Gly Ile Lys Arg Leu Phe Asp Phe
            20                  25                  30
Leu Ala Ser Ser Ile Ala Leu Ile Leu Leu Ser Pro Leu Phe Leu Trp
        35                  40                  45
Leu Ala Ile Lys Ile His Gly Glu Asp Gly Gly Pro Val Phe Tyr Ser
    50                  55                  60
Gln Ile Arg Ile Gly Lys Asp Glu Lys Pro Phe Arg Ile Tyr Lys Phe
65                  70                  75                  80
Arg Ser Met Val Val Asn Ala Glu Lys Leu Lys Lys Asp Leu Leu Glu
                85                  90                  95
Gln Asn Glu Val Glu Gly Ala Met Phe Lys Met His Asp Asp Pro Arg
            100                 105                 110
Ile Thr Lys Ile Gly Lys Phe Ile Arg Ala His Ser Leu Asp Glu Leu
        115                 120                 125
Pro Gln Leu Trp Asn Val Leu Ile Gly Asn Met Ser Leu Val Gly Pro
    130                 135                 140
Arg Pro Pro Leu Pro Asn Glu Val Glu Gln Tyr Thr Asp Tyr Asp Lys
145                 150                 155                 160
Gln Arg Leu Leu Val Lys Pro Gly Cys Ser Gly Leu Trp Gln Ala Thr
                165                 170                 175
Ser Arg Asn Ala Ala Asp Phe Ala Asp Met Val Gln Leu Asp Ile Glu
            180                 185                 190
Tyr Ile Asn Arg Ser Gly Val Phe Phe Asp Leu Trp Ile Ile Phe Lys
        195                 200                 205
Thr Ile Gly Val Met Phe His Pro Asn Gly Met
    210                 215

(2) INFORMATIONS POUR LA SEQ ID NO: 3:
(i) CARACTERISTIQUES DE LA SEQUENCE:
(A) LONGUEUR: 366 acides aminés
(B) TYPE: acide aminé
(C) NOMBRE DE BRINS: simple
(D) CONFIGURATION: linéaire
(ii) TYPE DE MOLECULE: protéine
(xi) DESCRIPTION DE LA SEQUENCE: SEQ ID NO: 3:

Met Lys Gly Asn Asn Lys Pro Arg Leu Leu Tyr Phe Val Glu Ala Met
1               5                   10                  15
Gly Gly Gly Val Phe Thr Tyr Ile Val Asp Leu Ala Asn Ser Leu Val
            20                  25                  30
Asp Asp Trp Asp Val Tyr Ile Gly Tyr Ala Met Arg Asn Gln Thr Pro
        35                  40                  45
Gln Asn Tyr Arg Glu Tyr Phe Asp Glu Arg Val His Leu Ile Glu Val
    50                  55                  60
Lys Asn Phe Ala Arg Ser Thr Ser Ile Thr Lys Ala Ile Lys Ala Gly
65                  70                  75                  80
Gln Glu Met Lys Arg Ile Ala Lys Ala Ile Arg Pro Asp Val Ile His
                85                  90                  95
Leu His Ser Ser Ile Ala Gly Ala Ile Gly Arg Val Val Phe Asn Thr
            100                 105                 110
Lys Lys Thr Pro Val Phe Tyr Thr Pro His Gly Tyr Ser Phe Leu Met
        115                 120                 125
Gln Gly Glu Ser Ser Lys Lys Arg Leu Ala Tyr Lys Leu Val Glu Gln
    130                 135                 140
Phe Cys Gly Lys Ser Gln Ala Thr Thr Ile Ala Cys Ser Pro Gly Glu
145                 150                 155                 160
Tyr Gln Glu Ala Leu Lys Val Ser Lys His Ala Val Glu Val Asp Asn
                165                 170                 175
Gly Ile Asn Ile Glu Gln Leu Gln Glu Leu Ile Asp Thr Thr Asp Ala
            180                 185                 190
Ser Lys Ile Asp His Tyr Asp Val Phe Thr Leu Gly Arg Ile Ser Val
        195                 200                 205
Gln Lys Asn Pro Asn Val Phe Asn Glu Val Ala Leu Lys Leu Ser Asn
    210                 215                 220
Leu Lys Phe Leu Trp Ile Gly Asp Gly Glu Leu Arg Ser Glu Leu Thr
225                 230                 235                 240
Ala Pro Asn Ile Thr Val Thr Gly Trp Leu Thr Arg His Glu Ala Leu
                245                 250                 255
Lys Tyr Ser Leu Asn Ser Asp Thr Phe Met Leu Thr Ser Leu Trp Glu
            260                 265                 270
Gly Leu Pro Met Ser Leu Leu Glu Ala Met Tyr Met Lys Lys Leu Cys
        275                 280                 285
Val Val Ser Asp Val Ile Gly Asn His Asp Val Ile Asn Asp Gly Val
    290                 295                 300
Asn Gly Tyr Val Cys Gln Thr Val Asn Asp Phe Tyr Asn Arg Ile Ser
305                 310                 315                 320
Met Ile Gly Gly Ala Arg Tyr Ser Leu Ile Asp Cys Ala Tyr Lys Asp
                325                 330                 335
Val Leu Glu Arg Tyr Asn Thr Lys Val Val Ser Lys Ala Tyr Asp Gln
            340                 345                 350
Leu Tyr Gln Ala Ala Ile Lys Asn Ser Glu Cys Phe Cys Leu
        355                 360                 365

(2) INFORMATIONS POUR LA SEQ ID NO: 4:
(i) CARACTERISTIQUES DE LA SEQUENCE:
(A) LONGUEUR: 375 acides aminés
(B) TYPE: acide aminé
(C) NOMBRE DE BRINS: simple
(D) CONFIGURATION: linéaire
(ii) TYPE DE MOLECULE: protéine

(xi) DESCRIPTION DE LA SEQUENCE: SEQ ID NO: 4:

Met Gln Lys Ile Arg Val Val His Val Ala Glu Ala Ala Gly Gly Val
1               5                   10                  15
Glu Arg Tyr Leu Tyr Ala Leu Leu Lys Asn Ser Asp Ser Gln Lys Ile
            20                  25                  30
Glu Asn Tyr Leu Ile Ala Ser Gln His Tyr Glu Met Gly Lys Phe Lys
        35                  40                  45
Lys Tyr Val Ser Gly Glu Tyr Gln Leu Gln Met Ala His Ala Ala Cys
    50                  55                  60
Phe Lys Asn Asp Lys Ala Thr Val Lys Gln Ile Lys Lys Thr Val Arg
65                  70                  75                  80
Gln Ile Lys Pro Asp Ile Val Tyr Ala His Ser Thr Lys Ala Gly Ala
                85                  90                  95
Leu Thr Arg Ile Ala Leu Met Gly Met His Ile Pro Val Ile Tyr Asn
            100                 105                 110
Pro His Gly Trp Ala Phe Asn Met Ala Gln Ser Lys Lys Lys Glu Leu
        115                 120                 125
Ala Tyr Arg Leu Ile Glu Lys Val Gln Ile Pro Phe Thr Lys Lys Val
    130                 135                 140
Val Cys Ile Ser Glu Ala Glu Met Gln Ser Ala Met Gln Lys His Ile
145                 150                 155                 160
Cys Ser Lys Asp Lys Ile Lys Ile Ile Pro Asn Gly Ile Asp Phe Asp
                165                 170                 175
Leu Leu Asp Lys Val Gln Pro Val Thr Arg Arg Asp Leu Gly Ile Leu
            180                 185                 190
Glu Asp Ala Phe Val Val Gly Gln Ile Gly Arg Leu Ser Glu Gln Lys
        195                 200                 205
Ser Pro Asp Val Phe Val Glu Met Ala Glu Lys Ile Lys Lys Glu Ile
    210                 215                 220
Pro Asn Ser Phe Phe Val Met Val Gly Asp Gly Asn Leu Glu Ala Glu
225                 230                 235                 240
Ile Arg Arg Leu Ile Lys Ser Lys Gly Leu Glu Asp Ser Phe Leu Ile
                245                 250                 255
Thr Gly Trp Val Asp Asn Pro Thr Gly Tyr Leu Asn Cys Phe Asp Val
            260                 265                 270
Ser Thr Leu Leu Ser Arg Trp Glu Gly Phe Gly Leu Ala Leu Val Glu
        275                 280                 285
Tyr Met Tyr Cys Gly Val Pro Leu Val Ser Thr Lys Val Asp Ala Ile
    290                 295                 300
Pro Tyr Val Val Asp Asp Gly Val Asp Gly Leu Leu Val Glu Pro Asp
305                 310                 315                 320
Asn Pro Tyr Glu Ala Ala Lys Ala Val Ile Arg Ile His Glu Asp Pro
                325                 330                 335
Ala Leu Ala Ala Lys Phe Val Ala Asn Gly Leu Ser Ile Ala Lys Asp
            340                 345                 350
Lys Tyr Asp Val Arg Arg Val Ala Lys Gln Thr Leu Lys Leu Tyr Tyr
        355                 360                 365
Lys Val Leu His Asn Leu Lys
    370                 375

(2) INFORMATIONS POUR LA SEQ ID NO: 5:
    (i) CARACTERISTIQUES DE LA SEQUENCE:
        (A) LONGUEUR: 268 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 5:

Met Pro Lys Val Ser Val Ile Met Ser Ile Tyr Asn Cys Lys Asn Tyr
1               5                   10                  15
Lys Ala Leu Glu Lys Ser Ile Glu Ser Ile Ile Asp Gln Thr Tyr Ser
            20                  25                  30

Asp Trp Glu Phe Ile Ile Tyr Asn Asp Gly Ser Glu Asp Asp Gly Lys
        35                  40                  45
Thr Ser Glu Tyr Leu Lys Lys Leu Gly Lys Arg Asp Ser Arg Ile Gln
    50                  55                  60
Ile Ile Asp Cys Pro Asn Asn His Gly Leu Ala Tyr Ala Lys Asn Glu
65                  70                  75                  80
Met Ile Lys Val Ala Arg Gly Asp Tyr Ile Thr Ala Gln Asp Asp Asp
                85                  90                  95
Asp Val Ser His Pro Met Arg Leu Ala Arg Glu Val Asp Phe Leu Asn
            100                 105                 110
Arg His Pro Glu Tyr Ala Phe Val Gly Thr Val Ala Lys Val Phe Asp
        115                 120                 125
Asn Ser Gly Thr Trp Gly His Tyr Gly Leu Lys Glu Lys Pro Thr Lys
    130                 135                 140
Asn Thr Phe Leu Trp Asn Ser Pro Phe Leu His Pro Ser Val Met Phe
145                 150                 155                 160
Arg His Glu Val Leu Asp Gln Val Asn Gly Tyr Arg Val Ala Lys Asp
                165                 170                 175
Thr Met Arg Ala Glu Asp Tyr Asp Leu Phe Phe Arg Leu Tyr Ala Lys
            180                 185                 190
Gly Tyr Lys Gly Tyr Asn Ile Gln Glu Asp Leu Tyr Glu Tyr Arg Ile
        195                 200                 205
Glu Asn Asn Pro Asn Lys Lys Tyr Arg Pro Met Ser Asp Arg Ile Gln
    210                 215                 220
Glu Ala Lys Val Arg Tyr Lys Gly Phe Lys Ser Phe Gly Met Leu Thr
225                 230                 235                 240
Lys Gly Ile Pro Tyr Ile Ile Lys Pro Ile Leu Ile Gly Leu Ile Pro
                245                 250                 255
Gln Lys Ile Phe Tyr Ile Ile Arg Lys Asn Arg Tyr
            260                 265

(2) INFORMATIONS POUR LA SEQ ID NO: 6:
    (i) CARACTERISTIQUES DE LA SEQUENCE:
        (A) LONGUEUR: 289 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 6:

Met Tyr Lys Asn Asp Leu Ser Phe Val Met Ser Lys Ile Leu Gly Arg
1               5                   10                  15
Leu Pro Ala Lys Arg Ile Ser Asp Lys Phe Asn Ile Tyr Arg His Gln
            20                  25                  30
Gln Ile Arg Lys Gln Leu Thr Pro Val Phe Asp Glu Ala Tyr Glu Thr
        35                  40                  45
Phe Thr Arg Tyr Asp Ser His Lys Val Asn Asp Thr Ile Trp Ile Phe
    50                  55                  60
Trp Trp Gln Gly Glu Asp Ser Met Pro Lys Val Val Lys Lys Cys Tyr
65                  70                  75                  80
Arg Ser Ile Gln Gln Asn Arg Gly Lys Lys Asn Ile Val Leu Ile Thr
                85                  90                  95
Gln Asp Asn Ile Lys Gln Tyr Thr Asp Ile Pro Lys Phe Ile Tyr Asp
            100                 105                 110
Lys Leu Asp Lys Gly Val Ile Thr Tyr Thr His Phe Ser Asp Ile Val
        115                 120                 125
Arg Ala Asn Leu Ile Lys Asn Asn Gly Gly Leu Trp Met Asp Ala Thr
    130                 135                 140
Leu Tyr Val Thr Ser Ser Leu Asp Asn Ile Asp Leu Lys Lys Leu Phe
145                 150                 155                 160
Tyr Cys Ser Gly Tyr Pro Ala Asp Thr Phe Asn Val Ser Phe Gly Arg
                165                 170                 175
Trp Thr Gly Phe Phe Met Gly Gly Pro Ser Gly Met Asp Leu Phe Ser
            180                 185                 190

Phe Met Asp Arg Leu Tyr Glu Val Tyr Trp Arg Asp His Glu Lys Leu
        195                 200                 205
Phe Asp Tyr Phe Leu Ile Asp Tyr Gly Leu Asp Tyr Ala Trp Lys Lys
    210                 215                 220
Asp Leu Ser Ser Phe Lys Ser Leu Glu Glu Ser Tyr Lys Gln Asn Asn
225                 230                 235                 240
Pro His Leu Phe Asp Leu Gln Gly Met Leu Asn Gln Pro Tyr Asp Asp
                245                 250                 255
Lys Glu Trp Lys Arg Ile Thr Ser Asp Thr Ser Val Phe Lys Leu Ser
            260                 265                 270
Tyr Lys Lys Lys Val Asp Phe Asp Lys Lys Glu Ser Tyr Tyr Asn Arg
        275                 280                 285
Leu

(2) INFORMATIONS POUR LA SEQ ID NO: 7:
    (i) CARACTERISTIQUES DE LA SEQUENCE:
        (A) LONGUEUR: 327 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 7:

Met Lys Leu Ser Ile Ile Val Pro Val Tyr Lys Val Glu Glu Tyr Leu
1               5                   10                  15
Lys Asp Cys Val Asn Ser Ile Leu Asn Gln Thr Phe His Asp Phe Glu
            20                  25                  30
Leu Ile Leu Val Asp Asp Gly Ser Pro Asp Ser Cys Pro Lys Ile Cys
        35                  40                  45
Asp Glu Tyr Ser Gln Lys Phe Asn Asn Val Lys Val Val His Lys Gln
    50                  55                  60
Asn Gly Gly Leu Ser Ser Ala Arg Asn Ala Gly Met Lys Val Ala Thr
65                  70                  75                  80
Gly Glu Tyr Ile Ser Phe Ile Asp Ser Asp Asp Tyr Leu Ala Ser Asn
                85                  90                  95
Met Tyr Glu His Val Phe Ser Ile Met Lys Lys Glu Cys Ala Asp Ile
            100                 105                 110
Val Val Val Gly Arg Cys Tyr Val Tyr Pro Asn Gly Ser Lys Lys Leu
        115                 120                 125
Arg Glu Lys Gln Asn Val Tyr Glu Val Met Asp Gly Pro Lys Ala Thr
    130                 135                 140
Ala Ile Met Asn Thr Ser Leu Leu Gly Tyr Phe Asp Ala Ala Ala Trp
145                 150                 155                 160
Asp Lys Val Tyr Lys Arg Ser Leu Phe Asn Asp Val Ser Tyr Pro Glu
                165                 170                 175
Gly Lys Leu Ser Glu Asp Trp Tyr Thr Thr Tyr Lys Val Phe Ala Lys
            180                 185                 190
Ala Asn Arg Ile Val Tyr Asp Ser Thr Pro Met Tyr Tyr Tyr Arg Gln
        195                 200                 205
Arg Gly Gly Ser Ile Thr His Thr Ser Ser Thr Val Asn Tyr Asp Ala
    210                 215                 220
Met Tyr Ala Ser Arg Glu Val Tyr Asp Phe Val Lys Lys Arg Gln Pro
225                 230                 235                 240
Glu Tyr Thr Ala Glu Ala Asn Phe Ala Tyr Val Phe Ser Arg Ile Gly
                245                 250                 255
Val Ile Asp Asn Leu Thr Val Gln Pro Thr Val Asp Lys Gln Lys Ile
            260                 265                 270
Arg Lys Ile Arg Asn Asp Met Lys Ala Asn Ile Lys Gln Leu Lys Lys
        275                 280                 285
Thr Asp Cys Phe Ser Lys Leu Ser Lys Lys Arg Lys Ile Gln Leu Phe
    290                 295                 300
Leu Ile Glu His Cys Leu Asn Ser Tyr Thr Ile Ile Phe Arg Arg Val
305                 310                 315                 320

Lys Ser Leu Gly Ile Ser Asp
                325

(2) INFORMATIONS POUR LA SEQ ID NO: 8:
    (i) CARACTERISTIQUES DE LA SEQUENCE:
        (A) LONGUEUR: 114 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 8:

Met His Ser Xaa Phe Asn Val Ser Xaa Xaa Asn Xaa Leu Thr Thr Leu
1               5                   10                  15
Leu Thr Ser Arg Ser Met Glu Met Asp Ala Asn Ser Val Ile Arg Glu
            20                  25                  30
Ser Gly Val Glu Asn Leu Ser Ile Leu Thr Ala Gly Pro Ile Pro Pro
        35                  40                  45
Asn Pro Ser Glu Leu Leu Ser Ser Lys His Met Leu Asp Leu Ile Glu
    50                  55                  60
Asp Leu Lys Gln Glu Tyr Asp Met Val Val Leu Asp Leu Ala Pro Ile
65                  70                  75                  80
Leu Asp Ala Gly Glu Thr Gln Gln Leu Thr Ser Ser Leu Asp Gly Thr
                85                  90                  95
Ile Leu Val Val Arg Gln Ser Tyr Ser Gln Lys Ser Ala Val Lys Arg
            100                 105                 110
Gly Ser

(2) INFORMATIONS POUR LA SEQ ID NO: 9:
    (i) CARACTERISTIQUES DE LA SEQUENCE:
        (A) LONGUEUR: 258 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 9:

Met Pro Val Val Asp Leu His Cys His Ile Leu Pro Gly Ile Asp Asp
1               5                   10                  15
Gly Ser Lys Ser Trp Glu Ala Ser Leu Lys Leu Ala Arg Ala Ala Val
            20                  25                  30
Ala Asp Gly Val Thr His Ala Leu Cys Thr Pro His Thr Leu Asn Gly
        35                  40                  45
Arg Tyr Thr Asn His Lys Gln Asp Val Ile Lys Leu Thr Glu Glu Phe
    50                  55                  60
Gln Asp Arg Ile Asp Gln Ala Gly Ile Pro Leu Thr Val Phe Pro Gly
65                  70                  75                  80
His Glu Val Arg Leu Ser Gly Gly Leu Thr Glu Ala Leu Asp Asn Asp
                85                  90                  95
Asp Ile Leu Phe Cys Asp Glu Glu Gly His Tyr Met Leu Leu Glu Leu
            100                 105                 110
Pro Ser Asn Glu Val Pro His Tyr Thr Lys Asn Met Val Tyr Glu Leu
        115                 120                 125
Thr Arg Arg Gly Ile Thr Pro Ile Val Val His Pro Glu Arg Asn Lys
    130                 135                 140
Lys Ile Leu Ala Asn Pro Gln Lys Leu Gln Glu Phe Leu Glu Met Gly
145                 150                 155                 160
Val Leu Val Gln Ile Thr Ala Ser Ser Tyr Thr Gly Leu Phe Gly Lys
                165                 170                 175
Glu Ile Glu Asp Cys Ser Arg Glu Phe Ile Lys Ala Gly Gln Cys Ala
            180                 185                 190

Phe Phe Ala Ser Asp Ala His Asp Leu Pro Arg Arg Gln Tyr Gln Leu
        195                 200                 205
Ser Glu Ala Leu Asp Lys Leu Ala Lys Glu Phe Gly Glu Asp Lys Lys
    210                 215                 220
Gln Lys Tyr Leu Asp Asn Ala Gln Ala Val Ile Asn Gly Asp Pro Val
225                 230                 235                 240
Tyr Leu Asn Trp Gln Pro Leu Pro Lys Lys Arg Lys Lys Phe Leu Gly
                245                 250                 255
Leu Phe

(2) INFORMATIONS POUR LA SEQ ID NO: 10:
    (i) CARACTERISTIQUES DE LA SEQUENCE:
        (A) LONGUEUR: 353 acides aminés
        (B) TYPE: acide aminé
        (C) NOMBRE DE BRINS: simple
        (D) CONFIGURATION: linéaire
    (ii) TYPE DE MOLECULE: protéine
    (xi) DESCRIPTION DE LA SEQUENCE: SEQ ID NO: 10:

Met Ser Val Tyr Phe Gly Leu Leu Ile Phe Cys Val Ile Ser Ser Met
1               5                   10                  15
Ile Gly Arg Gly Leu Thr Ile Asn Arg Phe Asp Ile Arg Ala Lys Ile
            20                  25                  30
Val Asp Val Leu Pro Phe Leu Ala Leu Phe Val Val Ser Ala Phe Arg
        35                  40                  45
Tyr Gln Val Gly Lys Asp Tyr Gly Ile Tyr Val Gly Thr Tyr Asp Leu
    50                  55                  60
Ile Lys Thr Gly Gln Gln Ala Arg Ile Asp Ile Phe Thr Tyr Trp Val
65                  70                  75                  80
Met Lys Val Met Ala Ser Leu Asn Leu Asp Val Gln Tyr Phe Phe Ile
                85                  90                  95
Val Ser Ser Phe Ile Val Asn Tyr Leu Ile Tyr Leu Ala Val Arg Asp
            100                 105                 110
Gln Ser Lys Asp Arg Thr Leu Ser Tyr Tyr Ile Tyr Ile Cys Gly Thr
        115                 120                 125
Leu Tyr Phe Ala Ser Met Asn Thr Val Arg Gln Met Met Ser Val Ala
    130                 135                 140
Met Phe Tyr Phe Ser Leu Lys Tyr Val Glu Ser Asn Asn Trp Lys Lys
145                 150                 155                 160
Tyr Phe Leu Cys Asn Leu Ile Gly Gly Leu Phe His Gln Thr Ala Phe
                165                 170                 175
Ile Phe Leu Pro Ile Tyr Phe Val Leu Lys Arg Tyr Met Gly Trp Lys
            180                 185                 190
Lys Tyr Leu Val Val Ile Ile Ile Ala Ile Ala Leu Lys Asn Pro Ile
        195                 200                 205
Val Asn Leu Leu Asn Gly Leu Ile Leu Asn Ser Lys Tyr Ala Met Tyr
    210                 215                 220
Val Thr Tyr Ser Asn Phe Ala Asn Thr Ala Trp Gly Gly Trp Lys Ile
225                 230                 235                 240
Ser Asn Phe Ile Asn Leu Ile Leu Phe Leu Ala Tyr Cys Leu Leu Leu
                245                 250                 255
Lys Asn Lys Asp Thr Glu Asp Ser Ile Tyr Thr Asn Ile His Phe Val
            260                 265                 270
Gly Val Leu Cys Ser Ile Leu Leu Leu Gly Ile Pro Gly Gly Asp Arg
        275                 280                 285
Ile Phe Leu Gly Phe Arg Phe Ile Glu Leu Leu Ser Val Pro Asn Leu
    290                 295                 300
Leu Tyr Lys Leu Lys Leu Arg Lys Ile Ser Tyr Ile Ile Leu Ser Leu
305                 310                 315                 320
Val Ile Ala Val Leu Tyr Leu Phe Tyr Phe Cys Tyr Thr Ile Gly Val
                325                 330                 335
Gln Asn Gly Asn Ser Val Leu Pro Tyr Val Thr Thr Leu Phe Ser Ile
            340                 345                 350
Asn

(2) INFORMATIONS POUR LA SEQ ID NO: 11:
(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire
(ii) TYPE DE MOLECULE: Autre acide nucl,ique
(A) DESCRIPTION: /desc = "amorces"
(xi) DESCRIPTION DE LA SEQUENCE: SEQ ID NO: 11:

GAYGARYTNC CNCARYTNWK NAAYGT 26

(2) INFORMATIONS POUR LA SEQ ID NO: 12:
(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire
(ii) TYPE DE MOLECULE: Autre acide nucl,ique
(A) DESCRIPTION: /desc = "amorces"
(xi) DESCRIPTION DE LA SEQUENCE: SEQ ID NO: 12:

CCANHRNCCN GTNANCCNGG YTT 23

## Revendications

1. Enzyme recombinée, susceptible d'être impliquée dans la biosynthèse de l'EPS ayant l'unité saccharidique répétitive 
β-D-Galp          α-L-Rhap          β-D-Galp
1                 1                 1
|                 |                 |
4                 3                 3
→3)-β-D-Galp(1→4)-α-D-Galp(1→2)-α-D-Galp(1→3)-β-D-Glcp(1→
 catalysant spécifiquement l'une des liaisons suivantes entre sucres:
- la liaison osidique β-1,3 entre le carbone 1 d'un D-Gal*p* activé et le carbone 3 du sucre présent à l'extrémité non-réductrice d'une chaîne saccharidique en formation, ladite chaîne étant composée d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique β-1,4 entre le carbone 1 d'un D-Gal*p* activé et le carbone 4 du sucre présent à l'extrémité non-réductrice d'une chaîne saccharidique en formation, ladite chaîne étant composée d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1;
- la liaison osidique α-1,3 entre le carbone 1 d'un L-Rha*p* activé et le carbone 3 du sucre présent à l'extrémité non-réductrice d'une chaîne saccharidique en formation, ladite chaîne étant composée d'une chaîne principale de 1 à 4 D-Gal*p* et/ou D-Glc*p* liés entre-eux par des liaisons osidiques α-1,2, α-1,3, β-1,4 et/ou β-1,3, et ledit sucre présent à l'extrémité non-réductrice étant lié à un seul autre sucre par son carbone 1.

2. Enzyme recombinée notamment selon la revendication 1, ayant la séquence en acides aminés choisie dans le groupe formé par les séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, et les séquences qui leur sont homologues.

3. ADN recombinée codant pour une enzyme selon la revendication 1 ou 2.

4. ADN selon la revendication 3, comprenant au moins un gène ayant une séquence nucléotidique choisie parmi les séquences délimitées dans la séquence SEQ ID NO:1 par les nucléotides 9-353, 516-1292, 1323-1982, 2074-3174, 3246-4373, 4511-5317. 5335-6204, 6709-7692, 7723-8784, et les séquences qui leur sont homologues.

5. Vecteur recombinant comprenant un ADN selon la revendication 3 ou 4.

6. Cellule comprenant, intégré dans son génome ou par le moyen d'un plasmide réplicable, un ADN recombiné selon la revendication 3 ou 4, et exprimant une enzyme recombinée fonctionnelle selon la revendication 1 ou 2.

7. Cellule selon la revendication 7, caractérisée en ce qu'il s'agit d'une bactérie lactique.

8. Procédé de production d'un EPS, dans lequel (1) on clone dans un vecteur un fragment d'ADN codant pour au moins l'une des enzymes selon la revendication 1 ou 2, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

9. Procédé selon la revendication 8, dans lequel le vecteur comprend en outre au moins une séquence promoteur fonctionnelle et au moins une séquence d'activation traductionnelle fonctionnelle, permettant l'expression des ADN codant pour au moins l'une des enzymes selon la revendication 1 ou 2.

10. Utilisation d'une enzyme recombinée selon la revendication 1 ou 2, ou d'un ADN selon la revendication 3 ou 4, pour la synthèse d'un EPS.
